# EUROPEAN PATENT APPLICATION

(11) **EP 1 712 567 A1**
(43) Date of publication of application: **18.10.2006**
(21) Application number: 05703965.3
(22) Date of filing: 21.01.2005
(51) Int. Cl.: C07K 17/02, G01N 33/53

(54) **PROTEIN IMMOBILIZATION METHOD AND QUANTIFICATION METHOD**

(30) Priority: 21.01.2004 JP 2004000504
(71) Applicant: Wako Pure Chemical Industries, Ltd., Osaka-shi, Osaka 540-8605 (JP); National Agriculture and Bio-oriented Research Organization, Ibaraki 305-0856 (JP)
(72) Inventor: KOHNO, Naoyuki, Amagasaki-shi, Hyogo 6610963 (JP); UEMORI, Hitoshi, Amagasaki-shi, Hyogo 6610963 (JP); NISHIBU, Takahiro, Amagasaki-shi, Hyogo 6610963 (JP); HIRAYASU, Kazunari, Amagasaki-shi, Hyogo 6610963 (JP); KOBAYASHI, Yoshiteru, Osaka-shi, Osaka 5408605 (JP); YOKOYAMA, Takashi, Tsukuba-shi, Ibaraki 3050047 (JP)
(74) Representative: von Kreisler, Alek
(86) International application number: PCT/JP2005/000737
(87) International publication number: WO 2005/070969

(57) **Abstract**

The present invention relates to a method for immobilizing a protein in a sample, which could not easily be immobilized by the conventional immobilization method, to a solid-phase; a method for quantitative determination of protein wherein an effect of inhibitory substance coexisting in a sample prepared using the immobilization method can be reduced; and a rapid and highly precise method for detecting an abnormal PrP and a method for determining BSE using the immobilization method as compared with the conventional method. The present invention provides: "a method for immobilizing a protein to a solid-phase comprising contacting the protein with the solid-phase having hydrophobic surface in the presence of a lower alcohol, and a halogenocarboxylic acid and/or a long chain alkyl sulfate, and an immobilizing reagent solution to be used therefor; a method for quantitative determination of protein comprising contacting a protein-staining solution with the solid-phase immobilized with a protein by the immobilization method, and determining a degree of color development generated thereby; an immunoblotting method wherein the solid-phase immobilized with a protein by the immobilization method is used; and a method for detecting an abnormal PrP a method for determining BSE by using the immobilization method."

## Description

### TECHNICAL FIELD

The present invention relates to a novel method for immobilizing a protein to a solid-phase, a method for quantitative determination of a protein using the novel method, an immunoblotting method and a reagent solution for immobilizing a protein. The present invention further relates to a method for detecting an abnormal prion protein and a method for determining prion disease (especially bovine spongiform encephalopathy, hereinafter designated as BSE).

### BACKGROUND ART

A conventional method for quantitative determination of a protein has been performed mainly by so called liquid-phase methods. These methods include a process for reacting a highly chemically reactive moiety of a protein with a protein reactive reagent solution in the solution (Lowry, O. H. et al., J. Biol. Chem., 193: 265-275 (1951); Smith, P. K. et al., Anal. Biochem., 150: 76-85 (1985)) or a process for measuring the protein based on the changes of the absorbance by reacting a protein with a specifically adsorbing pigment reagent (Bradford, M., Anal Biochem., 72: 248-254 (1976); Watanabe, N. et al., Clin. Chem., 32: 1551-1554 (1986)), to vary an absorbance of the solution. However, a biochemical sample generally contains many substances other than the protein such as non-proteinous biological substances, buffer, salts, antioxidant, chelating agent, sugars, organic solvent, artificial polymer and surfactant. As a result, accurate measurement of proteins cannot be performed. In order to solve such problem, a method for quantitatively determining a protein, so called a solid-phase method, has been developed. Namely, the protein is adsorbed to a solid-phase such as a membrane, and the inhibitory substances to the protein measurement such as above-mentioned coexisting substances are washed away, then the protein remained on the solid-phase surface is reacted with the reagent solution for measurement of the protein (Non-patent Document 1, Non-patent Document 2, Non-patent Document 3, Non-patent Document 4, and Non-patent Document 5).

Although the inhibitory substances can be removed from the membrane by the immobilization methods of a protein, the protein cannot be immobilized at a constant rate on the membrane and therefore, accurate quantitative determination of a protein cannot be performed. Consequently, the problem has been remaining unsolved.

Methods using immobilizing solution containing trichloroacetic acid or acetic acid, and acetic acid/methanol solution, which have a protein denaturation property and a charge neutralization property, are conventionally known. However, some types of protein cannot be immobilized sufficiently, and therefore, a quantitative measurement of the protein cannot be performed. Consequently, a development of novel methods for immobilization and a quantitative determination of a protein have been desired.

Prion disease represented by, for example, BSE, scrapie of sheep, human Creutzfeldt-Jakob disease, etc. is a disease caused by abnormalized prion protein (hereinafter prion protein is designated as PrP) with spongiformed brain tissues to develop neurologic symptom such as ataxia. Gene of PrP per se exists normally not only in bovine but also in mammals such as human, sheep and canine. PrP existing in the normal tissues of the normal individuals (an normal cellular PrP, hereinafter designated as normal PrP) is decomposed easily by proteinase K. However, PrP found in diseases such as BSE has specific characteristics having resistance to proteinase and physico-chemical treatment such as heating and ultraviolet irradiation (an abnormal isoform of the PrP, hereinafter this PrP is designated as abnormal PrP). In BSE, it is known that the abnormal PrP is accumulated in the brain to cause death of cells, thereby resulting sponge form brain to develop neurologic disorder.

Determination of prion disease is performed by detecting abnormal PrP, and ELISA and Western blotting method are known as the methods for detecting abnormal PrP.

The detection method of abnormal PrP by ELISA is performed as follows. The normal PrP in the sample to be tested is decomposed by, for example, enzymatic treatment, etc. to remain the abnormal PrP only, the abnormal PrP is reacted with an anti-PrP antibody (the primary antibody) bound to an ELISA plate, subsequently reacted with an anti-PrP antibody labeled with a labeling substance (the secondary antibody), then the labeling substance of the labeled anti-PrP antibody bound with the abnormal PrP is developed to coloring, and the color is measured. In this method, the normal PrP and other proteins have to be previously decomposed completely in order to be bound only the abnormal PrP with the anti-PrP antibody. However, there are possibilities that the normal PrP and other proteins cannot be completely decomposed actually, and remained such proteins may bound with the anti-PrP antibody (causing false positive determination), consequently specific detection of the abnormal PrP is difficult.

A detection method by Western blotting is performed by decomposing the normal PrP in the sample to be tested as same as in case of ELISA, and then subjected to electrophoresis. After that, the separated electrophoretic fractions are transferred to the sheet, a reagent which develops color by binding with the abnormal PrP is added, and such coloring is measured. In this method, since the protein in the sample to be tested can be classified into the group depending on molecular weights by performing the electrophoresis, only the abnormal PrP fraction can be detected separately, even when the normal PrP and other proteins co-exist in the sample to be tested. However, in Western blotting, several hours are required for obtaining results of the electrophoresis, and numbers of samples to be tested are limited for the electrophoresis.

Therefore, a rapid and highly specific method for detecting an abnormal PrP and a method for determining prion disease have been desired.

[Non-patent Document 1]: Kuno, H. et al., Nature, 1967, 215, p.974-975.
[Non-patent Document 2]: Gates, R., Anal. Biochem., 1991, 196, p.290-295.
[Non-patent Document 3]: Said-Fernandez, S. et al., Anal. Biochem. 1990, 191, p.119-126.
[Non-patent Document 4]: Ghosh, S. et al., Anal. Biochem., 1988, 169, p.227-233.
[Non-patent Document 5]: Lim, M. K. et al., Bio Techniques, 1996, 21, p.888-895.

### DISCLOSURE OF THE INVENTION

### PROBLEMS TO BE SOLVED BY THE INVENTION

The present invention has been accomplished on the basis of the above circumstances. It is an object of the present invention to provide: a method for immobilizing a protein in a sample which cannot easily be immobilized by the conventional immobilization methods, besides an effect of inhibitory substances which is coexisting in a sample can be reduced, and a quantitative determination /detection can be performed; a method for quantitative determination of a protein using the method; an immunoblotting method and a reagent solution used for immobilizing a protein; rapid and highly accurate method for detecting abnormal PrP by using the immobilization method; and a method for determining prion disease (especially BSE).

### MEANS FOR SOLVING PROBLEMS

The present invention has been made to attain the above object, and includes the following aspects. (1) A method for immobilizing a protein to a solid-phase, which comprises contacting the protein with the solid-phase having hydrophobic surface in the presence of a lower alcohol, a halogenocarboxylic acid and/or a long chain alkyl sulfate. (2) A method for quantitative determination of a protein, which comprises contacting the protein-staining solution with the solid-phase, immobilized with the protein by the method of the above (1), and the determining based on a degree of color development generated thereby. (3) A method for immunoblotting, comprising using the solid-phase to which the protein immobilized by the method of the above (1). (4) A method for detecting an abnormal PrP, which comprises immobilizing an abnormal PrP to a solid-phase by treating a sample to be tested containing the abnormal PrP by the method of the above (1); subsequently reacting with an antibody capable of binding to the abnormal PrP; measuring an amount of an antigen - antibody complex generated thereby, and detecting a presence of an abnormal PrP based on the results thereof. (5) A method for determining prion disease, which comprises detecting an abnormal PrP by the method of the above (4); and determining prion disease based on the results thereof. (6) An reagent solution for immobilizing a protein comprising a low alcohol, a halogenocarboxylic acid and/or a long chain alkyl sulfate. (7) A kit for detecting an abnormal PrP, which comprises as a constituent reagent: (i) an immobilizing reagent solution 1 containing a lower alcohol, and a halogenocarboxylic acid and/or a long chain alkyl sulfate; (ii) an immobilizing reagent solution 2 containing a lower alcohol, a halogenocarboxylic acid and a nonionic surfactant; and (iii) a labeled antibody capable to specifically bind to an abnormal PrP.

### EFFECTS OF THE INVENTION

According to the method for immobilizing a protein of the present invention, a protein can be immobilized sufficiently to a solid-phase as compared with conventional immobilization methods. And the quantitative determination of the protein can be performed which cannot be done accurately by conventional immobilization methods. Further, the sensitivity of immunoblotting that follow the immobilization becomes high when the method for immobilizing a protein of the present invention is performed.
Further, according to the method for detecting an abnormal PrP of the present invention, an advantageous effect can be expected, namely, the abnormal PrP can be detected rapidly and highly precisely, and rapid and highly precise determination of prion disease (especially BSE) can be achieved.

### BRIEF DESCRIPTION OF THE DRAWINGS

Fig. 1 shows the results obtained in Example 1, where a polyvinylidene difluoride membrane (a PVDF membrane) immobilized with a protein in each immobilization sample was stained with Pyromolex reagent solution, and an absorbance (a signal intensity) at 600 nm was measured.
Fig. 2 shows the results obtained in Example 2, where a PVDF membrane immobilized with a protein in each immobilization sample was stained with Pyromolex reagent solution, and an absorbance (a signal intensity) at 600 nm was measured.
Fig. 3 shows the results obtained in Example 3, where a PVDF membrane immobilized with a protein in each immobilization sample was stained with Pyromolex reagent solution, and an absorbance (a signal intensity) at 600 nm was measured.
Fig. 4 shows the results obtained in Example 4, where a PVDF membrane immobilized with a protein in each immobilization sample was stained with Pyromolex reagent solution, and an absorbance (a signal intensity) at 600 nm was measured.
Fig. 5 shows a calibration curve obtained in Example 5, where an ovalbumin (OVA) used as a protein sample was immobilized to a PVDF membrane and quantitative determination was made.
Fig. 6 shows a relative values obtained in Example 6, where these values were obtained based on the results of measurement on proteins by the immobilization method or the results of measurement on proteins by the liquid-phase method.
Fig. 7 shows calibration curves obtained in Example 7, where these calibration curves were obtained by performing the immobilization and the quantitative determination of a protein according to the present invention using IgG sample without containing sodium dodecyl sulfate (SDS) or IgG sample containing SDS as protein samples.
Fig. 8 shows results of immunoblotting obtained in Example 10, where A shows the results obtained by performing an immunodetection of the protein sample immobilized to the PVDF membrane by luminescent reaction and detected by exposing to an X-ray film, and B shows the results obtained by performing an immunodetection of the protein sample immobilized to the PVDF membrane by a coloring reaction and detected.
Fig. 9 shows the results obtained by performing immobilization and quantitative determination of protein according to the present invention using a diluted sample in Example 14, together with the results obtained by performing quantitative determination of protein by the conventional ELISA method in Comparative Example 4.

### EXPLANATION OF LETTERS OR NUMERALS

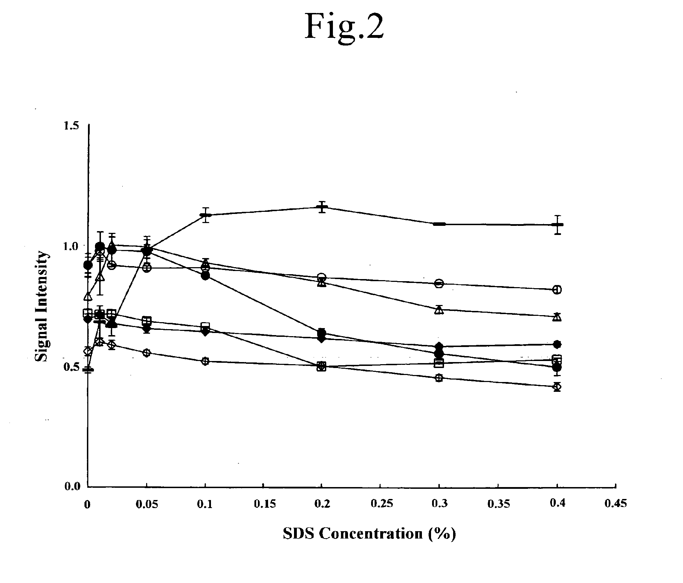

In Fig. 2, each symbol shows the result of a case using a protein sample containing: -Δ-: lysozyme; - - -: cytochrome c; -○-: IgG; -□-: fibrinogen; -●-: BSA; -◆-: OVA; -◇-: trypsin inhibitor, respectively.

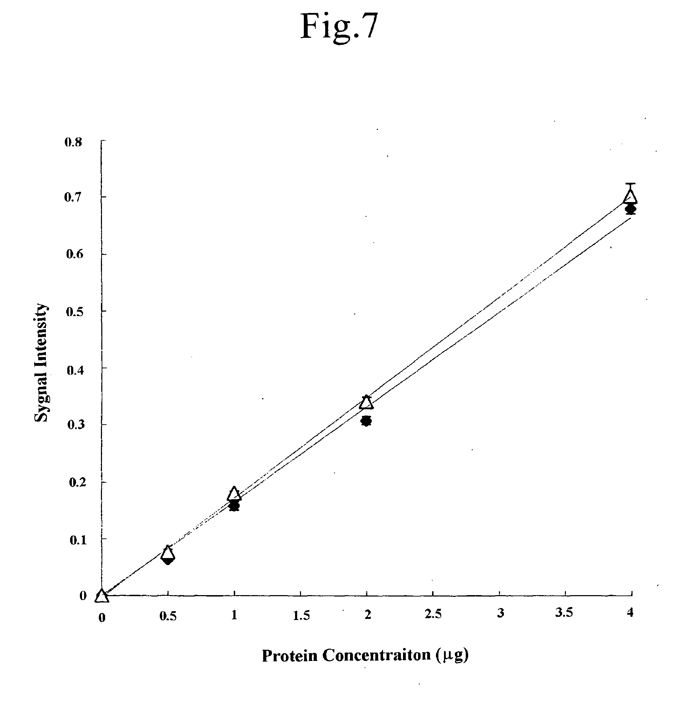

In Fig. 7, -◆- shows the results of a case using the IgG sample without containing SDS; and -Δ- shows the results of a case using the IgG sample containing SDS.

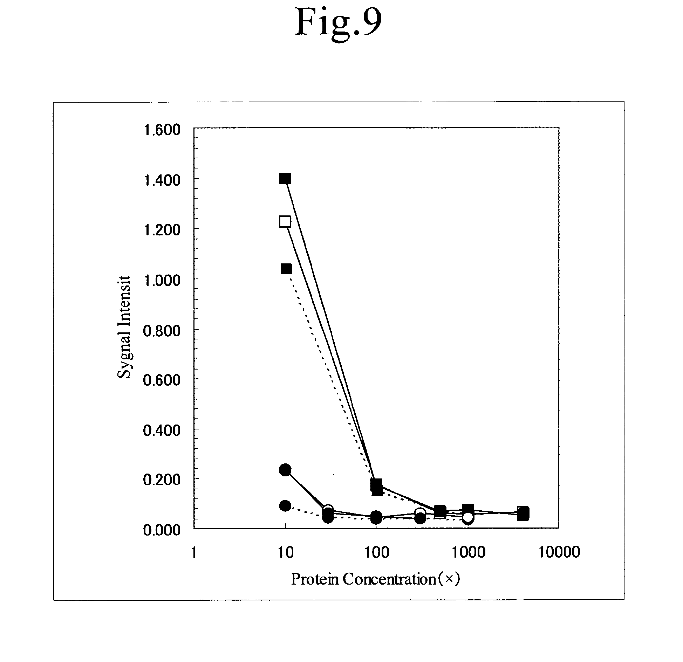

In Fig. 9, -□-, -■- and - -■- - show the results using sample No. 1, No. 2 and No. 3, respectively, obtained in Example 14, and -○-, -●- and - -●- - show the results of the cases using sample No. 1, No. 2 and No. 3, respectively, obtained in Comparative Example 4.

### BEST MODE FOR CARRYING OUT THE INVENTION

Thus, the present inventors have studied a factor by which immobilization of protein does not proceed well by the conventional solid-phase process, and then found that the biggest factor is that an objective protein cannot be immobilized to a solid-phase sufficiently (at a constant rate) in a stage to immobilize a protein to a solid-phase, and thereby quantitative determination of the protein can not be performed.
As a result of further study on a method for performing immobilization efficiently, the inventors have found that when immobilization of a protein is performed in the presence of lower alcohol such as ethanol and halogenocarboxylic acid such as trichloroacetic acid, a protein can be immobilized sufficiently to a solid-phase.

Further, the inventors have found that a long chain alkyl sulfate has a remarlable property for immobilization of a protein. Namely, it has been made apparent that when a protein is immobilized in the presence of a long chain alkyl sulfate together with a lower alcohol, or in the presence of a long chain alkyl sulfate together with a lower alcohol and a halogenocarboxylic acid, the reagents give some effect on the protein during suction filtration of the protein. As a result, the adsorption of the protein to a membrane is promoted, and coexisting substances such as inhibitors are excluded from the solid-phase surface of the membrane (to suppress retaining of inhibitors on the membrane). Such action of the long chain alkyl sulfate may occur, when the sample solution for immobilization containing a protein is allowed to put on the solid-phase surface such as microplate well.

Generally, surfactants such as nonionic surfactant have a property to inhibit adsorption of a hydrophobic substance. On the contrary, binding of a protein to a membrane is thought to occur by hydrophobic bonding. For this reason, surfactants have not been preferably used in the immobilization of proteins. Consequently, there are few reports discussing on an action of a surfactant against a protein and an interaction between the protein receiving the action and a solid-phase surface in detail. For this reason, it has not been known until now that a long chain alkyl sulfate as a kind of surfactant is effective for immobilization of a protein.

Accordingly, the idea, which a protein can be sufficiently immobilized to a solid-phase in the presence of a long chain alkyl sulfate together with a lower alcohol which had been conventionally used for immobilization of a protein, or together with a lower alcohol and a halogenocarboxylic acid, is found by the present inventors for the first time. Further, since a protein in a sample in the presence of a surfactant cannot be immobilized efficiently by the conventional immobilization method, quantitative determination of a protein in such a sample cannot be achieved, but according to the present invention, a protein in such a sample can be immobilized efficiently. As a result, an immobilization method for a protein of the present invention that is extremely effective and serves many uses has been completed.

Further, the present inventors considered an idea that rapid and highly precise determination of prion disease as compared with the conventional ELISA and Western blotting might be ensured by detecting an abnormal PrP using this immobilization method of a protein and determining a prion disease based on the results thereof. As a result, the inventors have, after extensive study, found that more accurate determination without false positive result as compared with the conventional ELISA can be performed, and a time required for determination can be shortened to 1/3 as compared with the conventional Western blotting by applying the conventional method for preparation of histological sample and the immobilization method for protein of the present invention, and then completed the present invention.

In the present invention, "a method for immobilizing a protein" means a method for immobilizing a protein to a solid-phase, and "measurement or quantitative determination of protein by a solid-phase method" means performing measurement or quantitative determination of a protein after immobilizing the protein to a solid-phase by the immobilization method of the present invention.

The lower alcohol of the present invention includes, for example, methanol, ethanol and propanol, etc., and ethanol or methanol is preferable.

The halogen atom of the halogenocarboxylic acid of the present invention includes, for example, bromine, fluorine, chlorine, etc., and chlorine is preferable. The carboxylic acid includes, for example, acetic acid, propionic acid, etc., and acetic acid is preferable. Examples of such halogenocarboxylic acid include, for example, trichloroacetic acid (TCA), trifluoroacetic acid (TFA), etc.

A long chain alkyl group of the long chain alkyl sulfate of the present invention is preferably an alkyl group of C₇₋₂₅, more preferably an alkyl group of C₈₋₁₅, and most preferably a dodecyl group. Sulfates are preferably sodium salt, potassium salt, etc. and among them sodium salt is preferable. Specific example of such long chain alkyl sulfate is, for example, sodium dodecyl sulfate (SDS), etc.

In the immobilization method of the present invention, a method to provide a protein with a lower alcohol, and a halogenocarboxylic acid and/or a long chain alkyl sulfate may be any one, so long as the protein can be coexisting with a lower alcohol, and a halogenocarboxylic acid and/or a long chain alkyl sulfate, when the protein is brought in contact with a solid-phase having hydrophobic surface.

For example, the method includes (1) a method for mixing a sample containing a protein, a solution containing a lower alcohol, and a solution containing a halogenocarboxylic acid and/or a long chain alkyl sulfate, and (2) a method for directly mixing a sample containing a protein, a lower alcohol, a halogenocarboxylic acid and/or a long chain alkyl sulfate, etc., but is not particularly limited.

Solution used for preparing a solution containing a lower alcohol, a solution containing a long chain alkyl sulfate and a solution containing a halogenocarboxylic acid includes, for example, purified water and buffer solution, etc. Buffering agent for buffer solution includes buffer agents commonly used in this field, for example, Good's buffer agent such as MOPS and HEPES, Tris buffer agent, phosphate buffer agent, Veronal buffer agent, borate buffer agent, etc. It is preferable to use purified water in order to avoid a possible effect on the immobilization and the measurement of proteins.

Preferable concentration of each reagent in an immobilization sample used when a protein is brought in contact with a solid-phase having hydrophobic surface, is as follows. A concentration of a lower alcohol is 30 to 50% (V/V), preferably 35 to 50% (WN). A concentration of a halogenocarboxylic acid is 0.08 to 10% (WN), preferably 0.5 to 5% (WN). A concentration of a long chain alkyl sulfate is 0.1 to 1% (WN), preferably 0.1 to 0.4% (WN).

The solid-phase having hydrophobic surface of the present invention includes, for example, a membrane having hydrophobic surface, a plate having hydrophobic surface, etc. Specific examples of the membrane having hydrophobic surface are, for example, a hydrophobic membrane such as polyvinylidene difluoride membrane (PVDF membrane), a nitrocellulose membrane and a filter paper, etc. Specific examples of the plate having hydrophobic surface are, for example, plastic plate such as one commonly used in ELISA, etc.

A method for bringing a protein in contact with the solid-phase having hydrophobic surface may be as follows. An immobilization sample containing a protein, a lower alcohol, and a halogenocarboxylic acid and/or a long chain alkyl sulfate prepared by the above method may be brought in contact with the solid-phase having hydrophobic surface. For example, the method includes dropping or coating the immobilization sample onto the solid-phase, etc.

When a hydrophobic membrane is used as the solid-phase, for example, a method of dropping the immobilization sample onto the hydrophobic membrane and allowing the immobilization sample to sink into the hydrophobic membrane by standing, a method of conventional filtration method, where the immobilization sample is filtered by suction through the hydrophobic membrane, or a centrifugal filtration method, etc. may be used.

The method for immobilizing a protein by the filtration method will be explained specifically by mentioning the method using a commercially available dot blotter or a slot blotter as follows.

First, a hydrophobic membrane such as PVDF membrane which is immersed in methanol then in distilled water, and if necessary, a filter paper which is immersed in distilled water, overlaid thereon, are set in this order in the dot blotter. Subsequently, an immobilization sample (maximum 400 µL) containing a certain amount of a protein, a lower alcohol, and a long chain alkyl sulfate and/or a halogenocarboxylic acid is applied to each well of the dot blotter. The dot blotter is vacuumed slowly at about 15 Kpa suction pressure by using a vacuum pump (for filtration) to adsorb the protein in the immobilization sample on the PVDF membrane. After the immobilization sample is completely vacuumed up, a washing solution is applied to each well and vacuumed up. Subsequently, the PVDF membrane is taken out from the dot blotter, and is placed on a paper towel or a filter paper to vacuum dry for over about 30 minutes.

Easiness on adsorption of a protein depends on a relationship between hydrophobicity of the protein and hydrophobicity of the solid-phase membrane surface. For example, a protein easily adsorbed under a condition can be adsorbed sufficiently even when it is vacuumed up rapidly. However, a degree of adsorption of a protein showing slight or weak adsorption property is greatly affected by suction rate.
Therefore, slow suction is generally preferable, in order to adsorb an objective protein. For example, a suction rate over no less than 10 minutes is preferable.

When a plate having hydrophobic surface is used as the solid-phase, for example, such a method may be performed that an immobilization sample is dropped or spread on the plate, followed by standing for natural drying.

For quantitative determination of a protein, the solid-phase immobilized with a protein may be subjected to a conventional quantitative determination of protein to measure an amount of protein in the sample.

The quantitative determination of a protein of the present invention can be performed by a known protein measuring method, namely, by immobilizing a protein to a solid-phase by the above method, then staining the immobilized protein by, for example, a method using a protein-staining solution such as amino black, pyrogallol red - molybdic acid complex (Pyromolex) solution, Bradford's method using Coomassie Brilliant Blue (CBB)-G250, and BCA method using bicinchoninic acid, etc., and then measuring a degree of color development generated thereby.

In a practical quantitative determination, a calibration curve is prepared by immobilizing, staining and measuring after a similar manner using protein samples containing known amount of protein, in advance measure a sample. A protein concentration in a sample is determined based on the calibration curve.

For, example, the quantitative determination method using Pyromolex coloring method is exemplified as follows. First, a protein in a protein sample is immobilized to a PVDF membrane by the method of the present invention, the obtained PVDF membrane is washed with purified water or a buffer solution such as phosphate buffered saline (PBS). After vacuum drying at room temperature for about 30 minutes if necessary, the membrane is immersed in a staining reagent solution containing Pyromolex for 20 to 35 minutes to develop coloring. Then, an absorbance of the developed color is measured at 600 nm by using a densitometer or a CCD camera, etc. Concentration of the protein is determined by applying the thus obtained absorbance to the calibration curve prepared in advance using protein samples containing known amount of protein by immobilizing and measuring the protein after a similar manner.

Incidentally, a washing treatment of the solid-phase may be optionally performed between each process of the immobilization of a protein and the measurement of the protein concentration, but is preferably performed. In this case, washing solution to be used is purified water and for example, a buffer solution such as PBS, etc.

With regard to the immunoblotting method of the present invention, a conventional immunoblotting method can be applied, in which determination/detection of the protein is performed by antigen-antibody reaction using an antibody or a labeled antibody against the protein, except for the immobilizing the protein to the solid-phase according to the method of the present invention. Since the protein can be effectively immobilized to the solid-phase by the immobilization method of the present invention, detection and analysis of the protein can be performed more precisely by using the immunoblotting method of the present invention as compared with the conventional method.

Example of a protein to be immobilized by the immobilization method of the present invention includes all of proteins, which can be immobilized by the conventional immobilization method, and specifically proteins contained in various body fluids such as blood, serum, plasma and spinal fluid, and samples derived from a living body such as urine, lymphocytes, blood cells and cells.

Specifically, such proteins include, but is not limited to, for example, enzyme such as lysozyme, cytochrome c and DNAase; antibody such as IgG, IgM and IgE; glycoprotein such as fibrinogen; serum protein such as bovine serum albumin (BSA), and human serum albumin (HAS); protein such as ovalbumin (OVA) and PrP; inhibitor such as trypsin inhibitor; and hormone such as insulin, etc.

The present invention is particularly useful on the point that basic protein such as cytochrome c, which had not been able to be immobilized sufficiently by means of the conventional immobilization method, can be immobilized and quantitatively determined.

In the method for immobilizing a protein of the present invention, since the upper limit of a concentration of a protein to be immobilized to a solid-phase is, for example, about 500 µg/cm² when a membrane is used for the solid-phase, and about 10 µg/cm² when a microplate is used for the solid-phase, an amount of protein in an immobilization sample is preferably prepared so that the amount of a protein does not exceed the maximum retention ability corresponding to a type of solid-phase to be immobilized.

Method for detecting an abnormal PrP of the present invention is a method comprising immobilizing the abnormal PrP to the solid-phase by treating a sample to be tested containing the abnormal PrP by the method for immobilizing a protein of the present invention, reacting with an antibody capable of binding to the abnormal PrP, measuring an amount of an antigen-antibody complex generated thereby, determining an amount of the abnormal PrP, and detecting PrP based on the results therefrom.

Method for preparing the sample to be tested containing the abnormal PrP used for the method for detecting the abnormal PrP of the present invention may be performed according to the method for preparing a sample derived from an animal tissues in the conventional determination method of BSE.

Namely, animal tissues containing an abnormal PrP to be detected are homogenized in a suitable buffer or a sugar solution, and the thus obtained homogenate is treated with proteinase K to digest a normal PrP. Subsequently, a protein precipitation agent is added to the reaction mixture to precipitate the abnormal PrP, the obtained precipitate is then dissolved in a suitable solvent (such as buffer and urea aqueous solution) and subjected to ELISA or Western blotting.

However, by treating the animal tissues containing the abnormal PrP to be detected by the method for preparation described below, the abnormal PrP can be extracted more efficiently from the animal tissues than the conventional method described above.

Namely, the method for preparation comprises:
1) a process for disrupting an animal tissue containing an abnormal PrP to be detected in the presence of a surfactant;
2) a process for removing an insoluble substance;
3) a process for decomposing a normal PrP by adding a decomposing enzyme into the supernatant;
4) a process for precipitating the abnormal PrP; and
5) a process for obtaining a solution of the precipitation after recovering the precipitate.

Examples of the surfactant used in the above process 1) include anion surfactant such as SDS, laurylbenzenesulfonic acid, deoxycholic acid, cholic acid and tris (hydroxymethyl) aminomethane dodecyl sulfate (Tris DS); and nonionic surfactant, for example, polyoxyethylene alkyl ether such as polyoxyethylene cetyl ether and polyoxyethylene lauryl ether, etc.; for example, polyoxyethylene alkyl phenyl ether such as polyoxyethylene octyl phenyl ether (Triton X-100, Rohm and Haas Co., Trade Name), etc.; for example, polyoxyethylene alkyl ester such as polyoxyethylene sorbitan monooleate, polyoxyethylene sorbitan monopalmitate, polyoxyethylene sorbitan monostearate and polyoxyethylene sorbitan triolate, etc.; for example, methyl glucamide derivative such as octanoyl-N-methyl-glucamide, nonanoyl-N-methyl-glucamide and decanoyl-N-methyl-glucamide, etc.; and for example, alkyl saccharide derivative such as n-octyl-β-D-glucoside. Among them, Triton X-100 and deoxycholic acid are preferable. These are used alone or in combination of two or more kinds.

Regarding to the method for disrupting animal tissues in the presence of the surfactant, so that the surfactant coexists when the animal tissue is disrupted, a surfactant or a homogenizing solution containing the same may be added to the animal tissues, or contrary, the animal tissues may be added to the surfactant or the homogenizing solution.

Concentration of the surfactant used in the process 1) is 0.015 to 15.6% (WN), and preferably 0.078 to 7.8% (WN), as a concentration in the homogenate when homogenized. Concentration in the homogenizing solution is 0.02 to 20% (WN), and preferably 0.1 to 10% (WN).

Incidentally, for example, buffering agents such as Tris buffer agent, phosphate buffer agent, Veronal buffer agent, borate buffer and Good's buffer agent, saccharides, salts such as NaCl, surfactant, antiseptic and protein other than the above surfactant may be contained in the homogenizing solution,

Method for removing insoluble substances in the above process 2) may be any method so long as the insoluble substances can be separated and removed, and a method using centrifugation is preferable because it is simple and easy way.

Decomposing enzyme to be used in the above process 3) may be any enzyme so long as the enzyme has a property to decompose a normal PrP but not decompose an abnormal PrP. For example, the enzyme includes proteinase K used in the conventional detection method for an abnormal PrP, etc. Any concentration and any condition can be used in the decomposing reaction, so long as the decomposing enzyme can decompose the normal PrP.

Examples of protein precipitation agent used in the above process 4) for precipitating the abnormal PrP can be an agent having a property to precipitate the abnormal PrP, and the agent used in the conventional detection method for the abnormal PrP can be used. For example, 3-butanol and a mixed solvent of 2-butanol and methanol, etc. can be used.

Method for recovering the precipitate in the above process 5) can be a method by which the precipitated abnormal PrP can be recovered by removing a supernatant, and a method by centrifugation is preferable because it is simple and easy way.

Further, in order to obtain a solution of the precipitate in the above process 5), the precipitate may be preferably dissolved in a suitable precipitate dissolving solution.

Solvent, which constitutes the precipitate dissolving solution, may be any one so long as the solvent does not have a property to inhibit the abnormal PrP being adsorbed or bound to the solid-phase , and includes, for example, purified water, and all of buffer solutions, etc. conventionally used in this field such as Tris buffer, phosphate buffer, Veronal buffer, borate buffer and Good's buffer. Value of pH of the buffer solution is not particularly limited, so long as the value is in a range not to inhibit the antigen-antibody reaction, and is preferably in a range of 5 to 9.

Further, a concentration of a buffering agent in the buffer solution is generally selected from a range of 10 to 500 mM, preferably 10 to 300 mM. Further, the dissolving solution may contain, for example, saccharides, salts such as NaCl, surfactant, antiseptic and protein, etc., so long as an amount thereof does not inhibit absorption or binding of the anti-PrP antibody to the solid-phase.

Further, surfactant, oxidant or protein denaturing agent such as urea, SDS, formic acid, guanidine thiocyanate, guanidine hydrochloride, trichloroacetic acid and phenol is required to coexist in the precipitate dissolving solution in order to inactivate the enzyme of the process 3) and to be inactivated the abnormal PrP (to inactivate its pathogenic activity= infectiveness). The concentration thereof is not less than 0.5% (W/V), preferably not less than 2% (W/V) as a concentration in the precipitate dissolving solution.

Incidentally, heat treatment as used in this field may also be performed in order to inactivate the enzyme.

Further, other reagents, instrumentations and treatment conditions (reaction temperature, reaction time, etc.) etc. used in each process of the processes 1) to 5) may be selected according to known methods for treating animal tissues which abnormal PrP should be detected as described above.

In a method for preparing a sample derived from an animal tissue in the conventional determination method of BSE, the animal tissue is homogenized and the allowed to a treatment with proteinase K. After that, an abnormal PrP is precipitated by adding a protein precipitation agent. However, no procedure is carried out for removing homogenate up to this stage from the beginning. For the reason, the thus obtained sample to be tested containing abnormal PrP contains large amounts of various proteins other than the abnormal PrP, and as a result, there may be a high risk for resulting false positive determination due to non-specific binding of the anti-PrP antibody occurred.

On the contrary, in the above process 1), cell membrane is solubilized by disrupting an animal tissue in the presence of a surfactant such as nonionic surfactant, and PrP existing in a form binding to cell membrane can be free from the cell membrane. Consequently, PrP does not precipitate together with tissue pieces, even when the process for removing precipitate is conducted in the subsequent process 2). Therefore, the solution containing PrP but not containing the animal tissue pieces can be obtained by recovering the supernatant. Since the obtained solution is treated in the process 3), a normal PrP can be decomposed efficiently as compared with the conventional direct enzymatic treatment of the homogenate, a sample to be tested containing the abnormal PrP but not containing the normal PrP can be obtained.

Further, since animal tissue pieces and proteins other than the abnormal PrP can be removed by repeating the procedures to remove substances other than the abnormal PrP in the processes 2) and 5), a risk of clogging of the solid-phase in the subsequent immobilization process can be avoided.

Further, in the processes 1) and 5), other proteins which are not to be determined and the normal PrP can be decomposed by providing a nonionic surfactant to the sample. On the contrary, since the abnormal PrP is a difficult protein to be denatured and decomposed, it is neither denatured nor decomposed even in the presence of a nonionic surfactant. Therefore, when the immobilization process to a solid-phase is performed, though the decomposed protein passes through the solid-phase, the abnormal PrP is not decomposed, and therefore it remains on the solid-phase. Consequently, the abnormal PrP can be immobilized efficiently to the solid-phase.

Method for immobilizing an abnormal PrP in a sample to be tested containing the abnormal PrP to the solid-phase can be performed by preparing the immobilization sample of an abnormal PrP containing a lower alcohol, and a halogenocarboxylic acid and/or a long chain alkyl sulfate using the sample to be tested containing the abnormal PrP prepared by the above method, and then the thus obtained sample is contacted with a solid-phase having hydrophobic surface, according to the method for immobilizing a protein of the present invention.

Preferable embodiments and specific examples of a lower alcohol, a halogenocarboxylic acid and a long chain alkyl sulfate to be used in the present method, method for preparing a solution containing the same and concentrations thereof in the solution, and method for providing a lower alcohol, and a halogenocarboxylic acid and/or a long chain alkyl sulfate with the sample to be tested, are as described above.

Specific examples of the solid-phase to be used in the method for contacting an immobilization sample of an abnormal PrP to a solid-phase having hydrophobic surface are as described above, and for example, a hydrophobic membrane such as PVDF membrane, nitrocellulose and filter paper, etc. is preferable.

Method for contacting an immobilization sample of an abnormal PrP to a solid-phase having hydrophobic surface and conditions for contacting are described above. Among them, a conventional filtration method with suction filtration through a hydrophobic membrane or centrifugal filtration method is preferable. By these methods, since the abnormal PrP to be determined is immobilized to a solid-phase and other proteins pass through the solid-phase, the process can be more effective.

In the present method, after the sample is contacted with a solid-phase in order to immobilize an abnormal PrP to the solid-phase, the solid-phase is allowed to stand for 1 minute to 30 minutes, preferably for about 10 minutes, then treatment with suction filtration is performed. Though conventional ELISA method requires about 75 minutes for standing in order to immobilize a protein on ELISA plate, such time can be greatly cut down by the present invention.

The method for immobilizing an abnormal PrP by the filtration method will be explained specifically by mentioning the method using commercially available dot blotter or slot blotter as follows.

First, a hydrophobic membrane such as a PVDF membrane which is immersed in methanol then in distilled water, and if necessary, a filter paper which is immersed in distilled water overlaid thereon, are set in the dot blotter. Subsequently, a sample for immobilization of an abnormal PrP (maximum 400 µL) containing an abnormal PrP, a lower alcohol, and a long chain alkyl sulfates and/or a halogenocarboxylic acid is applied to each well of the dot blotter. The dot blotter is vacuumed slowly at about 15 Kpa of suction pressure by using a vacuum pump (for filtration) to adsorb the abnormal PrP in the immobilization sample of the abnormal PrP on the PVDF membrane. After the solution is completely vacuumed up, a washing solution is applied to each well and vacuumed up.

The suction operation for immobilizing the abnormal PrP to a solid-phase may be performed under the conditions for sufficiently adsorbing the abnormal PrP, and the suction pressure may be about 2 Kpa to 30 Kpa. Time for suction and suction rate are not particularly limited.

After the abnormal PrP in the sample to be tested containing an abnormal PrP is bound to a solid-phase, substances in the sample to be tested such as a normal PrP and other proteins which are not bound to the solid-phase can be removed by the suction filtration, and the abnormal PrP to be determined can be immobilized efficiently to the solid-phase.

Incidentally, SDS, which possibly denature antibody, is remained on the solid-phase. Consequently, in the immobilization method of the present invention, optionally the solid-phase is washed with purified water or a buffer solution such as PBS after treatment of the immobilization as described above. In the method for immobilizing an abnormal PrP to a solid-phase, it is preferable to remove SDS in the sample by performing additional process for washing the solid-phase with a solution containing a nonionic surfactant after performing the process of the above suction filtration, and then to subject to subsequent detection process of the abnormal PrP. In these method, this method is effective one for performing immobilization of a protein which is difficult to denature like the abnormal PrP, since (1) other proteins have a possibility to denature by an action of the nonionic surfactant, but the abnormal PrP does not denature by such a treatment, and (2) nonionic surfactant has an action to uniformly spread the protein onto the solid-phase.

The solution to be used in the washing process may preferably contain a lower alcohol and a halogenocarboxylic acid in addition to a nonionic surfactant.

Preferable specific examples of a lower alcohol, a halogenocarboxylic acid and a nonionic surfactant to be used in the washing treatment are described above.

The preferable concentration of substances in the washing agent (designated as immobilizing reagent solution 2) in the washing treatment is a lower alcohol 80 to 50% (VN), preferably 30 to 50% (VN); a halogenocarboxylic acid 0.08 to 10% (WN), preferably 1 to 4% (WN); and a nonionic surfactant 0.01 to 10% (VN), preferably 0.04 to 2% (VN).

Upper limit of the concentration of a protein which can be immobilized to a solid-phase in the method for detecting the abnormal PrP of the present invention is as described previously.

In the method for detecting an abnormal PrP of the present invention, the conventional immunoblotting method performing determination / detection of an abnormal PrP according to the operational method for measurement of the known immunoassay method by using antibody or labeled antibody against the protein (for example, enzyme immunoassay (EIA), radioimmunoassay (RIA) and fluorescence immunoassay (FIA), etc.) can be applied except for immobilizing the abnormal PrP to a solid-phase according to the method of the present invention. Reagents used in that method such as buffering agent, coloring agent, fluorescent substance, enzyme, substrate and radioisotope can be selected from substances used in the known immunological assay method.

An antibody to be used in the method for detecting an abnormal PrP of the present invention is not particularly limited so long as the antibody has a property to bind to an abnormal PrP, and the antibody may one having a property to bind to a normal PrP.

Further, origin of the antibody is not particularly limited, and includes, for example, the antibody having the properties described above derived from human, rabbit, equine, bovine, goat, sheep, rat, etc.

Further, the anti-PrP antibody may be any one of polyclonal antibody or monoclonal antibody. For example, a commercially available monoclonal antibody or any monoclonal antibody having a property as described above and produced by a known method utilizing a cell fusion technique or a gene recombinant technology (Eur. J. Immunol., 6, 511 (1976)) can be used. With regard to polyclonal antibody, a commercially available polyclonal antibody can be used, or any polyclonal antibody obtained from antisera of animal by a known method (e.g. methods described in "Tanpakushitu Seisei-hou" by Robert K. Scopes, Springer Verlag Tokyo K.K., 1985, p. 37-179) can be used. These are optionally used alone or in combination thereof.

Further, these antibodies can be used after being digested with an enzyme such as pepsin and papain into F(ab')₂, Fab' or Fab, if necessary.

Incidentally, considering specificity of an antibody having uniform property, monoclonal antibody is more preferable than polyclonal antibody.

In the method for detecting an abnormal PrP of the present invention, labeling substance to be used for labeling an anti-PrP antibody includes but not limited to, for example, peroxidases such as peroxidase derived from horse radish, enzyme such as alkaline phosphatase, β-galactosidase, microperoxidase, glucose oxidase, glucose-6-phosphate dehydrogenase, malic dehydrogenase and luciferase which are used in EIA, radioisotopes such as ^{99m}Tc, ¹³¹I, ¹²⁵I, ¹⁴C and ³H which are used in RIA, fluorescent substances such as fluorescein, dansyl, fluorescamine, coumarin, naphthylamine, or derivatives thereof which are used in FIA, luminescent substance such as luciferin, isoluminol, luminal and bis(2,4,6-triflorophenyl)oxalate and other luminescent substances, substances having an absorption in ultraviolet light such as phenol, naphthol, anthracene or derivatives thereof, and a substances which have characteristic as spin-labeling agent represented by oxyl grou-containing compounds such as 4-amino-2,2,6,6-tetramethylpiperidine-1-oxyl, 3-amino-2,2,5,5-tetramethylpiperidine-1-oxyl and 2,6-di-t-butyl-α-(3,5-di-t-butyl-4-oxo-2,5-cyclohexadiene-1-ilydene)-p-tryloxyl, etc.

Among them, enzymes such as peroxidase are preferable, because they are easy-to-use.

Further, method for binding (labeling) the labeling substance mentioned above to the anti-PrP antibody may be conducted after a per se known one so far conducted generally in per se known EIA, RIA, FIA etc. (e.g. Yuichi Yamamura "Ikagaku Jikken Koza" Vol.8, 1 st ed., NAKAYAMA-SHOTEN Ltd., 1971; Akira Kawano "Zusetsu Keikokotai" 1st ed., Soft Science, Inc., 1983; and Eiji Ishikawa, Tadashi Kawai and Kiyoshi Miyai "Koso Men-eki Sokuteiho" 2nd ed., IGAKU-SHOIN Ltd., 1982, etc.). Since an anti-PrP antibody labeled with the labeling substance is available in the market, it can be used.

A solvent for preparing a solution containing a labeled anti-PrP antibody may be any solvent, so long as the solvent does not have an inhibitory property for adsorbing or binding an anti-PrP antibody to the solid-phase. For example, the solvent includes purified water and any buffer solution commonly used for assay methods utilizing an antigen-antibody reaction such as Tris buffer, phosphate buffer, Veronal buffer, borate buffer and Good's buffer, etc. Range of pH of the buffer solution is not particularly limited so long as the range of pH does not suppress an antigen-antibody reaction, and generally a range of pH 5 to 9 is preferable.
A concentration of the buffering agent in these buffer solutions is generally selected from a range of 10 to 500 mM, preferably 10 to 300 mM. Further, for example, saccharides, salts such as NaCl, a surfactant, an antiseptic and a protein, etc. may be contained in this solution, so long as an amount thereof does not inhibit adsorption or binding of an anti-PrP antibody to a solid-phase.

Method for measuring an amount of the labeling substance in an antigen-antibody complex formed by the antigen-antibody reaction depends on types of the labeling substance, and can be performed based on a property detectable by any of methods in each labeling substance. For example, when the labeling substance is enzyme, the measurement is carried out according to a conventional method of EIA, for example, the method described in Tsunehiro Kitagawa, Toshio Nanbara, Akio Tsuji, and Eiji Ishikawa "Koso Men-eki Sokuteiho," an extra issue No.31 of Tanpakushitsu Kakusan Koso, pp.51-63, KYORITSU-SHUPPAN Ltd., published on Sep.10, 1987 etc. When the labeling substance is a radioisotope, the measurement is carried out by using a measuring instrument such as Geiger-Müller (GM) counter, a liquid scintillation counter, a well-type scintillation counter, an HPLC counter or the like which is chosen depending on the kind and intensity of radiation ray emitted by said radioisotope (see, for example, Yuichi Yamamura, "Ikagaku Jikken Koza," vol.8, 1st ed., NAKAYAMA-SHOTEN Ltd., 1971), according to a conventional method of RIA. When the labeling substance is fluorescent substance, the measurement is carried out according to a conventional method of FIA using a measuring instrument such as a fluorophotometer, for example, the method described in Akira Kawano, "Zusetsu Keikokotai," 1st ed., Soft Science Inc., 1983 etc. When the labeling substance is a luminescent substance, the measurement is carried out according to a conventional method using a measuring instrument such as a photo counter, etc., for example, the method described in Tsunehiro Kitagawa, Toshio Nanbara, Akio Tsuji, and Eiji Ishikawa, "Koso Men-eki Sokuteiho," an extra issue No.31 of Tanpakushitsu Kakusan Koso, pp.252-263, KYORITSU-SHIUPPAN Ltd., 1987, etc. When the labeling substance is a substance having an absorption in ultraviolet light, the measurement is carried out by a conventional method using a measuring instrument such as a spectrophotometer. When the labeling substance is a substance which have characteristics as spin-labeling agent, the measurement is carried out according to a conventional method using an electron spin resonance apparatus described, for example, the method described in Tsunehiro Kitagawa, Toshio Nanbara, Akio Tsuji, and Eiji Ishikawa, "Koso Men-eki Sokuteiho," an extra issue No.31, of Tanpakushitsu Kakusan Koso, pp.264-271, KYORITSU-SHUPPAN Ltd., published on Sep.10, 1987, etc.

More specifically, for example, in a case when the labeling substance is an enzyme, a known method such as the method for measuring the amount of color that generates by the reaction of the enzyme with the coloring reagent such as a substrate that generates color by the enzyme reaction can be performed.

A coloring reagent to be used for such purpose is one conventionally used as coloring agent in this field, and includes, for example, trimethylbenzyl piperazine (TMB), tetramethylbenzidine, o-phenylenediamine, o-nitrophenyl-β-D-galactoside, 2,2-azino-bis(3-ethylbenzthiazoline-6-sulfonate) (ABTS), N-ethyl-N-sulfopropyl-m-anisidine (ADPS) and p-nitrophenyl phosphate.

Further, to terminate the coloring reaction, a method for terminating a reaction generally used in this field can be utilized, including, for example, addition of an enzyme such as 1 to 6 N sulfuric acid can be applied.

All of a labeled antibody, a coloring reagent and other reagents, conditions of assay (such as reaction temperature, reaction time, wavelength for measurement and measuring apparatus, etc.) to be used in the method for detecting an abnormal PrP of the present invention can be sufficiently selected from those in the known immunoassay methods described above, and can be performed according to the operational method of the known immunoassay method, and as for autoanalyzer, spectrophotometer, etc., those conventionally used in this field can be used without exception.

The method for detecting an abnormal PrP of the present invention will be explained specifically below.

Namely, for example, a POD labeled anti-PrP antibody is applied to a solid-phase immobilized with an abnormal PrP obtained by the method of the present invention, allowed to stand for about 10 minutes, and suction filtered to remove the labeled PrP antibody which is not bound to PrP. Then a washing agent is applied and suctioned. This washing operation can be performed optionally for several times. Then, the coloring reagent solution containing TMB is applied and reacted for about 30 minutes. After filtration by suction is performed to remove the coloring reagent solution, a stop solution is added to terminate the reaction. Absorbance at 450 nm is measured with a microplate reader, and the like.
Since an abnormal PrP can be immobilized efficiently to a solid-phase by performing the immobilization method of the present invention, detection and analysis of the abnormal PrP according to the immunoblotting method of the present invention can be performed with higher sensitivity than the conventional method for detection of an abnormal PrP such as ELISA and Western blot technique.

Animal tissue, whose abnormal PrP to be detected in the method for detecting an abnormal PrP of the present invention, includes tissues of central nervous system such as medulla, cerebellum, spinal cord and others, reticular lymphoid tissues such as lymph node and bone marrow derived from animals. Originating animals include human, bovine, sheep, hamster and mouse, etc.

Method for determining prion disease of the present invention includes, for example, a method performed by setting out a cut off value, etc. A case of determining BSE is shown below as an example thereof.

(1) First, the above method for detecting a PrP is performed by using a PrP (normal type) as a sample, and an amount (e.g. absorbance, the same as above) of the labeling substance of the labeled anti-PrP antibody is measured, then a mean value and a standard deviation (SD) are calculated. The mean value + XSD is set as a cut off value, wherein X is generally selected from integers of 2 to 5. The X becomes smaller, the rate of specimen determined to be BSE becomes higher. However, taking the measurement into consideration, if the value of X becomes smaller, the rate of false positive is thought to increase also. For this reason, when specimens are measured actually, X is desirably set to about 5.
   Separately, a labeling amount of the labeled anti-PrP antibody is measured using a sample to be tested derived from a specimen which BSE is to be determined by the same method mentioned above to obtain a value of the specimen.
   If the value of the specimen is lower than the cut off value, the specimen is determined to be negative for prion disease. On the contrary, if the value of the specimen is equivalent to or higher than the cut off value, the specimen is determined to be positive for prion disease. This is one method.
(2) Alternatively, the above method for determining a PrP is performed by using a PrP (normal type) as a sample, and a labeling amount of the labeled anti-PrP antibody is measured, then a mean value and a standard deviation (SD) are calculated to obtain Mean value + XSD, wherein X has the same meaning as above.
   Separately, a labeling amount of the labeled anti-PrP antibody is measured using a solution without containing a PrP as a sample by the same method mentioned above (negative control) by the same method to obtain a mean value. The mean value of the amount of labeling of the negative control is added to the value obtained previously above, i.e. [(mean value in the case of specimen of normal PrP) + XSD] + (mean value of the negative control) to set the resulting value as a cut off value.
   Further, an amount of labeling of the labeled anti-PrP antibody is measured using a sample to be tested derived from a specimen which BSE is to be determined by the same method mentioned above to obtain a value of the specimen.
   If the value of the specimen is lower than the cut off value, the specimen is determined to be negative for prion disease. On the contrary, if the value of the specimen is equivalent to or higher than the cut off value, the specimen is judged to be positive for prion disease. This is the another method.

PrP to be used for obtaining the cut off value can be a PrP obtained by purification from animal tissue, or a recombinant PrP prepared by a genetic engineering technique.

Prion disease, which can be determined by the method for determining prion disease of the present invention, includes in particular BSE.

A reagent solution for immobilizing a protein of the present invention may be any reagent so long as the reagent containing a lower alcohol, and a halogenocarboxylic acid and/or a long chain alkyl sulfate of the present invention, and specific examples are as described before.

Further, a concentration of the lower alcohol can be a concentration to provide 30 to 50% (VN), concentration of the halogenocarboxylic acid can be a concentration to provide 0.1 to 10% (W/V), and concentration of a long chain alkyl sulfate can be a concentration to provide 0.1 to 1% (W/V), in the reagent solution for immobilizing a protein when the protein is immobilized to a solid-phase. More preferably, the concentration is 35 to 50% (V/V) for a lower alcohol, 0.5 to 5% (W/V) for a halogenocarboxylic acid and 0.1 to 0.4% (W/V) for a long chain alkyl sulfate.

Further, the reagent solution for immobilizing a protein of the present invention may additionally contain, for example, salts, a chelating agent, etc. so long as they do not provide any detrimental effect on the immobilization of a protein to a solid-phase or subsequent quantitative determination of protein.

A kit for detecting an abnormal PrP of the present invention comprises (1) an immobilizing reagent solution 1 containing a lower alcohol, and a halogenocarboxylic acid and/or a long chain alkyl sulfate; (2) an immobilizing reagent solution 2 containing a lower alcohol, and a halogenocarboxylic acid and a nonionic surfactant; and (3) a labeled antibody capable of binding to an abnormal prion protein, as constituent reagents, and specific embodiment thereof is as described above.

Further, when the labeled antibody constituting the above kit is an enzyme labeled antibody, the kit further comprises a substrate, which can generate a detectable signal by a reaction with the enzyme, as a constituent reagent. Preferable embodiments and specific examples are as described above.

In the following, the present invention is further explained in details referring the Example, and the present invention is not limited thereto by any means.

### EXAMPLES

### Example 1

### Preparation of sample and reagent solution

(1) Protein sample
   Ovalbumin (hereinafter designated as OVA, derived from chicken egg albumin, Wako Pure Chemical Industries, Ltd.), hemoglobin (derived from bovine blood, Wako Pure Chemical Industries, Ltd.), IgG (derived from bovine, Wako Pure Chemical Industries, Ltd.), cytochrome c (derived from horse cardiac muscle, Wako Pure Chemical Industries, Ltd.), and lysozyme (derived from chicken egg albumin, Wako Pure Chemical Industries, Ltd.), were weighed and dissolved in purified water to prepare a solution of 250 µg/mL each as the protein sample.

(2) Immobilizing reagent solution
   Each reagent was dissolved in purified water to prepare the following immobilizing reagent solutions. Among them, the immobilizing reagent solutions 3 to 5 are the immobilizing reagent solutions of the present invention.
   In each reagent, ethanol (Wako Pure Chemical Industries, Ltd., Reagent grade), trichloroacetic acid (hereinafter designated as TCA. Wako Pure Chemical Industries, Ltd., for chemical use) and sodium dodecyl sulfate (hereinafter designated as SDS, Wako Pure Chemical Industries, Ltd., for chemical use) were used.
   Control: purified water;
   Immobilizing reagent solution 1: 0.2% (WN) SDS;
   Immobilizing reagent solution 2: 0.2% (WN) SDS, 2.5% (WN) TCA;
   Immobilizing reagent solution 3: 0.2% (WN) SDS, 45% (V/V) ethanol;
   Immobilizing reagent solution 4: 0.2% (WN) SDS, 2.5% (WN) TCA, 45% (VN) ethanol;
   Immobilizing reagent solution 5: 2.5% (WN) TCA, 45% (VN) ethanol.

(3) Immobilization sample
   A protein sample in an amount of 20 µL (protein: 5 µg) and 300 µL of a given immobilizing reagent solution were mixed to prepare the immobilization sample. Final concentration of each reagent in the immobilization sample (concentration in contacting with PVDF membrane, hereinafter the same as above) is as described below.
   Immobilization sample 1: 0.19% (WN) SDS;
   Immobilization sample 2: 0.19% (WN) SDS, 2.34% (WN) TCA;
   Immobilization sample 3: 0.19% (WN) SDS, 42.2% (VN) ethanol;
   Immobilization sample 4: 0.19% (WN) SDS, 2.34% (WN) TCA, 42.2% (VN) ethanol;
   Immobilization sample 5: 2.34% (WN) TCA, 42.2% (VN) ethanol.

Immobilization and determination of protein
   A hydrated polyvinylidene difluoride membrane (PVDF membrane, Millipore Corp., Immobilon P^{SQ}, 0.1 µm) was set up on the dot blotter ADVANTEC DP-96 (Advantec MSF Inc.). The immobilization sample in an amount of 320 µL was applied on the PVDF membrane, and then suctioned and filtered for 10 minutes at 15 KPa (10 cmHg) by using a vacuum pump (Biocraft Inc.). Subsequently, 300 µL of phosphate buffer (PBS), pH 7.4, was applied, suctioned and filtered in the similar manner as above to wash the PVDF membrane. The PVDF membrane was taken out, dried in vacuo, and then treated with Pyromolex reagent solution (Protein Assay Rapid Kit wako, Wako Pure Chemical Industries, Ltd.) to develop color, and absorption at 600 nm (signal intensity) was measured by using a densitometer SHIMADZU CS-9000 (Simadzu Corp.).

### Results

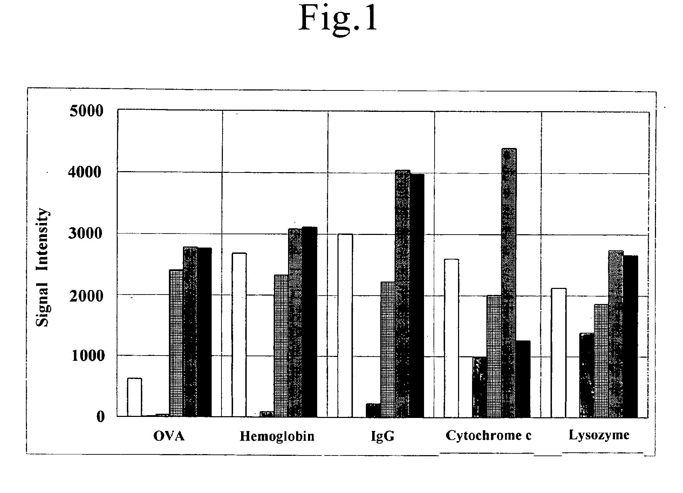

The results are shown in Fig. 1. In Fig. 1, each bar indicates the result using the corresponding immobilization sample as shown below.

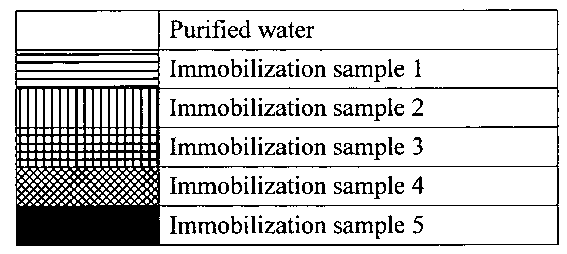

As is clear from Fig. 1, when the immobilization sample 1 containing only SDS as the reagent was immobilized to the membrane, the signal intensity could not be measured, at all (Immobilization sample 1). When the immobilization sample 2 containing SDS and TCA was used, the signal intensity was slightly increased (measurable), but the signal intensity as high as the control (in the case using purified water, that is, conventional immobilization method) could not be measured.
Contrary, when the immobilization sample 3 containing SDS and ethanol was used, the signal intensity equivalent to or higher than the control could be measured.
When the immobilization sample 5 containing TCA and ethanol was used, the signal intensity far higher than the control could be measured in all cases except for the case of immobilizing cytochrome c.
When the immobilization sample 4 containing TCA, ethanol and SDS was used, the signal intensity far higher than the control could be measured in all cases including the case of immobilizing cytochrome c.
From the above facts, it is understood that the immobilization rate of a protein to a membrane can be improved spectacularly by the immobilization method of the present invention which is conducted in the presence of a lower alcohol, a halogenocarboxylic acid and/or a long chain alkyl sulfate, as compared with the conventional immobilization method using only water or a buffer solution.
Further, it is also understood that a protein can be immobilized by the immobilization method of the present invention even when the sample containing SDS in advance is used, since the signal intensity equivalent to or higher than the control could be measured by using the immobilization samples 3 and 4.

### Example 2

### Preparation of sample and reagent solution

(1) Protein sample
   Lysozyme, cytochrome c, IgG, fibrinogen (derived from human plasma, Wako Pure Chemical Industries, Ltd.), BSA (bovine serum albumin, Wako Pure Chemical Industries, Ltd.), OVA, trypsin inhibitor (derived from soy bean, Wako Pure Chemical Industries, Ltd.), and hemoglobin were weighed and dissolved in purified water to prepare a solution of 250 µg/mL each as the protein sample.
   These proteins have a wide range of the isoelectric points of pI 4.0 to 11.4, and the molecular weights of 12,000 to 150,000 (refer to Kubo et al., Tanpakushitu in "Seikagaku Handbook" Maruzen K.K., 54-57 (1984)).
(2) Immobilizing reagent solution
   Solutions containing 2.5% (WN) TCA, 45% (VN) ethanol and given concentrations (0 to 0.4% (WN)) of SDS were prepared by dissolving each reagent in purified water to use as the immobilizing reagent solutions.
(3) Immobilization sample
   A predetermined protein sample in an amount of 20 µL (protein 5 µg) and 300 µL of the immobilizing reagent solution were mixed to prepare the immobilization sample (the final concentration of TCA was 2.34% (WN) and the final concentration of ethanol was 42.2% (VN)).
   Immobilization and determination of protein
   The protein in each immobilization sample was immobilized to the PVDF membrane, washed and stained, and measured the absorbance (signal intensity) at 600 nm by using a densitometer in the same manner as in Example 1.

### Results

### Results are shown in Fig. 2.

In Fig. 2, each symbol shows the result of the case using the protein sample containing: -Δ-: lysozyme; - - -: cytochrome c; -○-: IgG; -□-: fibrinogen; -●-: BSA; -◆-: OVA; and -◇-: trypsin inhibitor, respectively. An abscissa axis indicates SDS concentration of in the immobilizing reagent solution. Further, in Fig. 2, the bar in each point indicates ±SD.
As is clear from Fig. 2, when the proteins were immobilized to the PVDF membrane in the presence of SDS, the signal intensities in almost all proteins were once increased, but when the SDS concentration was increased to 0.1% (WN) or more, the signal intensities showed a tendency to be maintained at a constant level. From these results, the immobilization rate of proteins in the sample to the PVDF membrane may be kept constant by maintaining SDS concentration in the immobilizing reagent solution at a concentration not less than 0.1% (WN) (final concentration in the immobilization sample is not less than 0.09% (WN)).
From the viewpoint of CV value (CV value = standard variation / mean value (%)) showing the variation of data, it is understood that when the concentration of SDS in the immobilizing reagent solution is lower than 0.1 % (W/V), every protein shows the large CV value, that is, the signal intensity is not maintained stably. Contrary, it is indicated that when the SDS concentration in the immobilizing reagent solution is 0.1% (W/V) or more, CV value is relatively small, that is, a value of the signal intensity is maintained stably within such a range of concentration as described above. The results are considered to indicate an amount of a protein immobilized to a solid-phase membrane. Further, although data are not shown, this is confirmed to be reproducible.

Generally, a long chain alkyl sulfate such as SDS is said to show an action to destroy a structure of a protein even in a considerably low concentration such as 0.0025% (WN), and to generate a difference in reactivity to a stain (dye) bound to the protein depending on a degree of the structure destruction (Orsonneau, J-L et al., Clin. Chem., 35, 2233-2236 (1989)). Consequently, it is inferred that the difference in reactivity causes here again to show a rapid change such as an increase in signal intensity when the immobilizing reagent solution with a SDS concentration lower than 0.1% (W/V) is used, and since it is on the way of changes, CV value (<15%) becomes larger. Further, it is suggested that there is a possibility of changing (not to stay constant) the immobilization rate of the protein in the sample to the PVDF membrane under the condition of SDS concentration lower than 0.1 % (W/V).
From the above, in Fig. 2, it is inferred that when the absorbance (signal intensity) is stabilized without being influenced by the change in SDS concentration, the rate of immobilization of the protein to the PVDF membrane may become constant.

Therefore, based on the results of Fig. 2, immobilization was performed with the immobilizing reagent solution containing 0.1% (W/V) of SDS using BSA as a protein sample, and then the measurement was performed and the obtained signal intensity was set as 100 (reference value). And, immobilizations and measurements were performed using various proteins as protein samples with the immobilizing reagent solution containing 0.1% (W/V) of SDS, 0.2% (W/V) of SDS, 0.3% (W/V) of SDS, and 0.4% (W/V) of SDS, in the similar manner mentioned above to obtain the signal intensity, the relative values of the signal intensity against the reference value were calculated.

The results obtained when the SDS concentration was varied from 0.2% (W/V) to 0.4% (W/V) are shown in Table 1, and the results obtained when the SDS concentration was varied from 0.1% (W/V) to 0.4% (W/V) are shown in Table 2. The results include: An average value of CV values at each point (mean CV value (%)); a mean value of relative values at each point; an average value of absolute deviations between the points (mean absolute deviation); and a value indicated by percentage obtained by dividing the mean absolute deviation by the mean value (rate of variability (%) between points). A rate of variability between the points means a value, in which the change of signal intensity accompanied to the change of SDS concentration is calculated as a rate of variability. It is indicated that as number of the value becomes smaller, signal intensity (result of measurement) becomes more stable without being influenced by the SDS concentration.

**Table 1**

| Protein | Mean CV value (%) | Mean value | Mean absolute deviation | Rate of variability between points (%) |
|---|---|---|---|---|
| BSA | 4.9 | 65.6 | 7.0 | 10.7 |
| Trypsin inhibitor | 2.6 | 53.1 | 4.0 | 7.5 |
| Fibrinogen | 1.8 | 58.5 | 0.8 | 1.4 |
| OVA | 1.5 | 68.3 | 1.4 | 2.0 |
| Hemoglobin | 1.7 | 71.9 | 4.6 | 6.4 |
| IgG | 0.8 | 96.5 | 1.9 | 1.9 |
| Cytochrome c | 1.9 | 127.1 | 3.5 | 2.7 |
| Lysozyme | 1.8 | 87.4 | 6.4 | 7.4 |
| | Mean CV value 2.1 | | | Mean rate of variability between points 5.0 |

**Table 2**

| Protein | Mean CV value (%) | Mean value | Mean absolute deviation | Rate of variability between points (%) |
|---|---|---|---|---|
| BSA | 3.9 | 74.2 | 13.87 | 18.7 |
| Trypsin inhibitor | 2.5 | 54.4 | 4.62 | 8.5 |
| Fibrinogen | 1.5 | 62.7 | 6.38 | 10.2 |
| OVA | 1.3 | 69.6 | 2.32 | 3.3 |
| Hemoglobin | 1.7 | 68.6 | 5.94 | 8.7 |
| IgG | 0.8 | 98.3 | 3.17 | 3.2 |
| Cytochrome c | 2.1 | 127.4 | 2.93 | 2.3 |
| Lysozyme | 1.8 | 92.0 | 9.48 | 10.3 |
| | Mean CV value 2.0 | | | Mean rate of variability between points 8.1 |

As a result from Table 1, when SDS concentration is between 0.2% (W/V) to 0.4% (W/V), for example, fibrinogen, OVA, IgG and cytochrome c were most stable in the rate of variability between points, showing 1.4 to 2.7%. It is by no means inferior as compared with the mean CV values (0.8 to 1.9%) of these proteins. These results indicate that the variability of signal intensity by the effect of SDS concentration is small.
From the results of Table 2, three proteins except for fibrinogen show the rates of variability between points of 2.3 to 3.3% even in SDS concentration between 0.1 to 0.4% (W/V), and therefore, small variation of the signal intensity caused by an effect of SDS concentration can be known.
In fibrinogen, lysozyme and BSA, which shows the rate of variability between points over 10% at SDS concentration of 0.1 to 0.4% (W/V), it is understood that a stable result can be obtained when SDS concentration is limited within 0.2 to 0.4% (W/V).
From the above facts, since the rate of variability between points can be stabilized at a SDS concentration not less than 0.1% (W/V), it is understood that when a protein is immobilized by using the immobilizing reagent solution containing SDS within such concentration range, quantitative determination of the protein in the sample can be achieved exactly.

### Example 3. Examination of lower alcohol

Immobilization and determination of protein samples were performed where methanol was used in place of ethanol as the lower alcohol.

### Preparation of sample and reagent solution

(1) Protein sample
   BSA, OVA, hemoglobin, IgG, cytochrome c and lysozyme were weighed and dissolved in purified water to prepare a solution of 250 µg/mL each as the protein samples.
(2) Immobilizing reagent solution
   The following immobilizing reagent solutions were prepared by using purified water.
   Immobilizing reagent solution 1: 0.2% (WN) SDS, 2.5% (WN) TCA;
   Immobilizing reagent solution 2: 0.2% (WN) SDS, 2.5% (WN) TCA, 45% ethanol;
   Immobilizing reagent solution 3: 0.2% (WN) SDS, 2.5% (WN) TCA, 45% methanol (Wako Pure Chemical Industries, Ltd., Reagent grade).
(3) Immobilization sample
   Each protein sample in an amount of 20 µL (protein 5 µg) and 300 mL of a given immobilizing reagent solution were mixed to prepare the immobilization sample 1, the immobilization sample 2 and the immobilization sample 3. The final concentration of each reagent in the immobilization sample is SDS 0.19% (WN), TCA 2.34% (WN), ethanol 42.2% (VN), and methanol 42.2% (VN).
   Immobilization and determination of protein
   The protein in each immobilization sample was immobilized to a PVDF membrane, washed, stained, and measured an absorbance (signal intensity) at 600 nm by using a densitometer in the same manner as in Example 1.

### Results

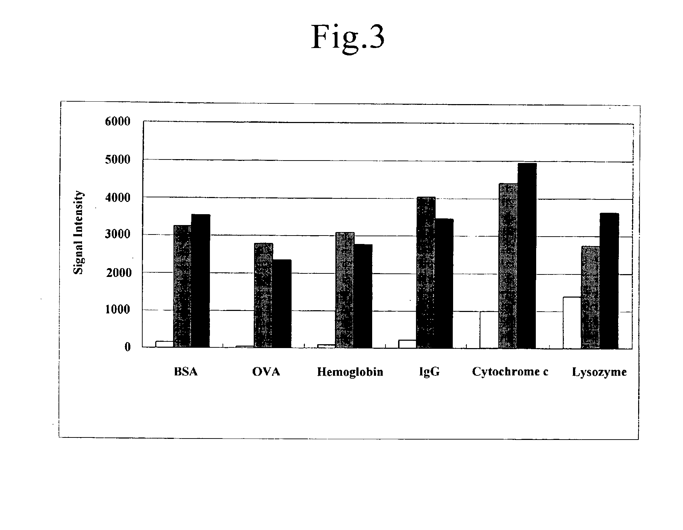

The results are shown in Fig. 3. In Fig. 3, each bar indicates the result using the corresponding immobilization sample as shown below.

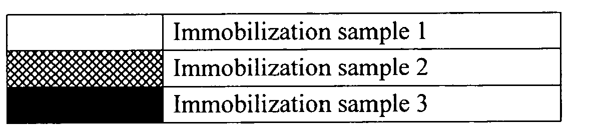

As is clear from Fig. 3, when an immobilization sample prepared by using an immobilizing reagent solution containing methanol was used, an almost same level of signal intensity as the case using ethanol could be obtained, and it is understood that immobilization of the protein to the PVDF membrane can be achieved.

### Example 4. Examination of halogenocarboxylic acid

Immobilization and determination of protein sample were performed where trifluoroacetic acid (hereinafter designated as TFA) was used in place of TCA as the halogenocarboxylic acid.
Preparation of sample and reagent solution
(1) Protein sample
   BSA, IgG and lysozyme were weighed and dissolved in purified water to prepare a solution of 250 µg/mL each as the protein samples.
(2) Immobilizing reagent solution
   The following immobilizing reagent solutions were prepared by using purified water.
   Immobilizing reagent solution 1: 0.2% (WN) SDS, 45% (V/V) ethanol;
   Immobilizing reagent solution 2: 0.2% (WN) SDS, 45%(V/V) ethanol, 2.5% (WN) TCA;
   Immobilizing reagent solution 3: 0.2% (WN) SDS, 45%(V/V) ethanol, 2.5% (WN) TFA (Wako Pure Chemical Industries, Ltd., reagent grade).
(3) Immobilization sample
   Each protein sample in an amount of 20 µL (protein 5 µg) and 300 mL of a given immobilizing reagent solution were mixed to prepare the immobilization sample 1, the immobilization sample 2 and the immobilization sample 3. The final concentration of each reagent in the immobilization sample is SDS 0.19% (W/V), TCA 2.34% (W/V), TFA 2.34% (W/V), and ethanol 42.2% (V/V).
   Immobilization and determination of protein
   The protein in each immobilization sample was immobilized to a PVDF membrane, washed, stained, and measured an absorbance (signal intensity) at 600 nm by using a densitometer in the same manner as in Example 1.

### Results

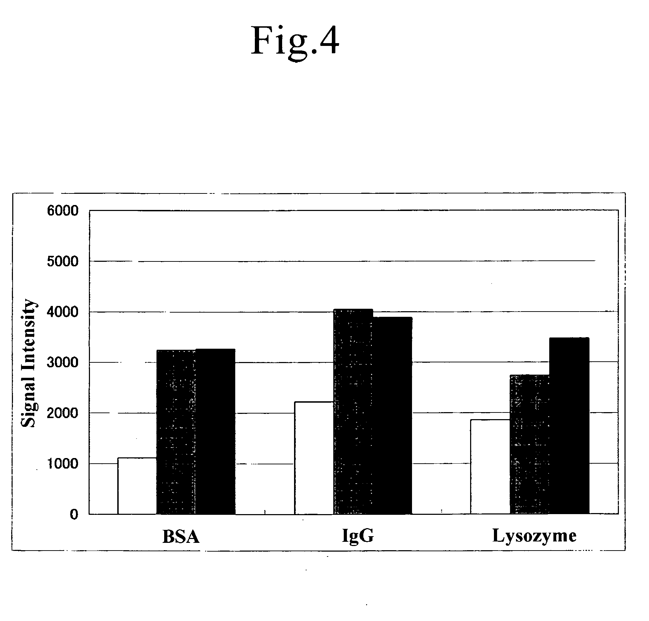

The results are shown in Fig. 4. In Fig. 4, each bar indicates the result using the corresponding immobilization sample as shown below.

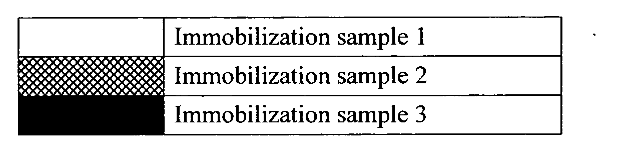

As is clear from Fig. 4, when an immobilization sample prepared by using the immobilizing reagent solution containing TFA was used, a signal intensity of almost same level to or higher than that of the case using TCA could be obtained, and it is understood that immobilization of the protein to the PVDF membrane can be achieved.

### Example 5. Preparation of calibration curve

### Preparation of sample and reagent solution

(1) Protein sample
   OVA was dissolved in purified water so that the concentrations thereof became 2 to 20 µg/20 µL to prepare the protein samples.
(2) Immobilizing reagent solution
   Each reagent was dissolved in purified water so that a concentration thereof became 0.2% (VN) SDS, 2.5% (VN) TCA and 45% (VN) ethanol to prepare the immobilizing reagent solutions.
(3) Immobilization sample
   Each protein sample in an amount of 20 µL (protein 5 µg) and 300 µL of an immobilizing reagent solution were mixed to prepare the immobilization samples. The final concentration of each reagent in the immobilization sample is SDS 0.19% (WN), TCA 2.34% (WN), and ethanol 42.2% (VN).
   Immobilization and determination of protein
   The protein in each immobilization sample was immobilized to a PVDF membrane, washed, stained, and measured an absorbance (signal intensity) at 600 nm by using a densitometer in the same manner as in Example 1.

### Results

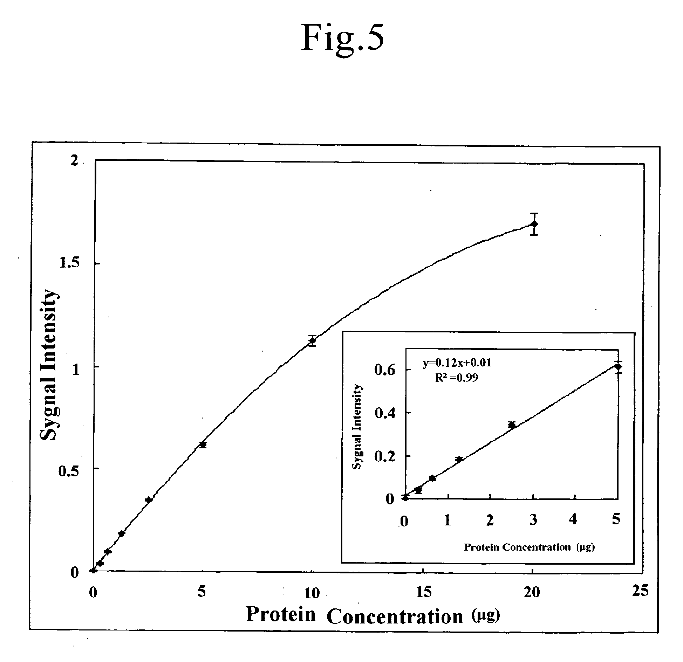

Based on the results obtained above, a calibration curve showing a relationship between an amount of protein (µg) and signal intensity was obtained.
The results are shown in Fig. 5. In Fig. 5, each bar on each plot shows ±2SD.
The results obtained from the calibration curve were: measuring range: 0.2 to 20 µg/protein sample; coefficient of concordance: not less than 0.99; and mean CV: 1.9%.
Further, linearity was obtained within a range of a protein concentration of 0.2 to 5 µg/protein sample. A regression line equation and correlation coefficient within this range obtained by a statistical processing of the measurement results are as follows.
Regression line equation: y = 0.12x + 0.01
Correlation coefficient (R²): 0.99
x: amount of protein
y: signal intensity
As is clear from Fig. 5, since the calibration curve exhibiting good linearity was obtained by immobilizing the OVA with the PVDF membrane and measuring an amount of the protein according to the method of the present invention, it was understood that the quantitative determination of OVA (protein) concentration with high accuracy was able to be achieved according to the immobilization method of the present invention.
In addition, although data are not shown, as the results of the measurements similarly performed for other proteins determined in Example 2, calibration curves with linearity similar to the result of OVA was obtained, and it was found that the quantitative determination of these proteins can be performed.

### Example 6

### Preparation of sample and reagent solution

(1) Protein sample
   A solution of BSA, trypsin inhibitor, fibrinogen, OVA, hemoglobin, IgG, cytochrome c and lysozyme of 5 µg/20 µL each was dissolved in purified water to prepare the protein samples.

### Determination of protein by solid-phase method

An immobilizing reagent solution containing SDS 0.2% (W/V), TCA 2.5% (W/V) and ethanol 45% (VN) was prepared by using purified water. The thus prepared protein sample in an amount of 20 µL and 300 µL of the immobilizing reagent solution were mixed. The protein in the immobilization sample was immobilized in the same manner as in Example 1 to a PVDF membrane by using 320 µL of the immobilizing reagent solution, washed, stained, and measured an absorbance (signal intensity) at 600 nm using a densitometer. The final concentration of each reagent in the immobilization sample was, SDS: 0.19% (W/V), TCA: 2.34% (W/V) and ethanol: 42.2% (VN), respectively. Determination of protein by the liquid-phase method
Pyromolex solution in an amount of 1 mL was added to 20 µL of the protein sample prepared above, and incubated at room temperature for 20 minutes to measure an absorbance at 600 nm.

### Results

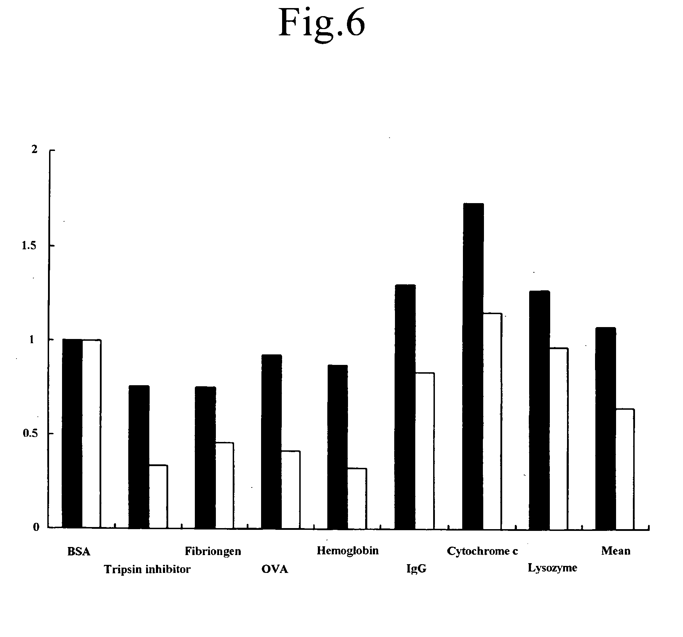

An immobilization sample containing SDS 0.2% (W/V) and BSA as a protein sample was prepared. Then, BSA was immobilized and measured to obtain an absorbance value (signal intensity), and the thus obtained value was set to 1 as a reference value. Each protein was immobilized and measured an absorbance value in the same manner as above, then a relative absorbance value of each protein to the reference value was calculated. The results are shown in Fig. 6. Similarly, BSA was used as a protein sample and measured by the liquid-phase method to obtain an absorbance value, the thus obtained value was set to 1 as a reference value. Each protein was measured in the same manner as in the liquid-phase method, and a relative absorbance value to the reference value was calculated. The results are also shown in Fig. 6.

In Fig. 6, each bar shows the above relative value based on the result of measurement for each protein by the following methods.

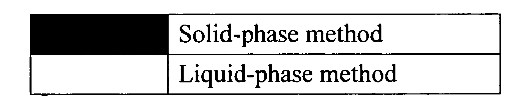

As is clear from Fig. 6, in the measurement by the liquid-phase method, even when a concentration of protein is same, a relative absorbance value to BSA is largely different depending on kind of the protein. For example, the value is about 0.46 in the case of fibrinogen.
Contrary, when the determination is made by the solid-phase of the present invention, the relative value of the absorbance (signal intensity) of fibrinogen to BSA is 0.75. The result shows a smaller difference than the case of the liquid-phase method. This can be referable to almost all proteins determined thereby.
Further, a relative value of average absorbance of all proteins determined to an absorbance of BSA, which is set as 1, is 0.65 for the case of the liquid-phase, whereas 0.94 for the case of the solid-phase of the present invention. From this, it is found that a quantification error caused by a difference in kind of proteins is improved. This might be due to that basic amino acid such as cytochrome c and lysozyme, which can not react with the staining solution in the liquid-phase method, can be in a state capable to bind to the staining solution by trapping the protein in a denatured state with the membrane, and the protein can be immobilized efficiently with the immobilizing reagent solution of the present invention, as a result, more exact measurement result can be obtained.

### Example 7

### Preparation and immobilization of sample

A sample of IgG (amount of protein: 0 to 4 µg/20 µL) and a sample of IgG containing 2% SDS (amount of protein: 0 to 4 µg/20 µL) were prepared by using purified water. Separately, an immobilizing reagent solution containing 0.25% (WN) SDS, 2.5% (WN) TCA and 45% (V/V) ethanol was prepared by using purified water. The protein sample in an amount of 20 µL and 300 µL of an immobilizing reagent solution were mixed to prepare 320 µL of an immobilizing reagent solution. The protein in the immobilization sample was immobilized to a PVDF membrane by using the obtained 320 µL of immobilizing reagent solution in the same manner as in example 1, washed, stained, and measured an absorbance (signal intensity) at 600 nm using a densitometer.

A final concentration of each reagent in the immobilization sample obtained by using IgG sample without containing SDS is SDS: 0.23% (WN), TCA: 2.34% (WN) and ethanol: 42.2% (VN). A final concentration of each reagent in the immobilization sample obtained by using IgG sample containing 2% SDS is SDS: 0.36% (WN), TCA: 2.34% (WN) and ethanol: 42.2% (VN).

### Results

Calibration curves showing a relationship between an amount of protein (µg) and signal intensity in the case using IgG sample without containing SDS and IgG sample containing 2% SDS were prepared based on the obtained results, respectively,
The results are shown in Fig. 7. In Fig. 7, -◆- shows the result of the case using the IgG sample without containing SDS; and -Δ- shows the result of the case using the IgG sample containing SDS. A bar in each plot shows ±SD.
As is clear from Fig. 7, both calibration curves coincide well with each other.
From the above results, it is understood that the quantitative determination of a protein can be performed by immobilizing the desired protein to a solid-phase by the immobilization method of the present invention, even when SDS is present in a protein sample, without being influenced by SDS by immobilizing the protein.

### Example 8

Immobilization and determination of a protein of the present invention was performed using various protein samples containing SDS, and an effect of SDS on the determination was compared with the case of determination in the liquid-phase method. Preparation of sample and reagent sample
(1) Protein sample
   Protein samples (BSA, trypsin inhibitor, fibrinogen, hemoglobin, OVA, cytochrome c, lysozyme, IgG, and trypsin (Wako Pure Chemical Industries, Ltd)), 250 µg/mL each, containing 0.0% (WN) SDS (control), 0.2% (W/V) SDS or 2% (WN) SDS were prepared by using purified water.
   Determination of protein by the immobilization method
   An immobilizing reagent solution containing 0. 1% (W/V) SDS, 2.5% (WN) TCA and 45% (V/V) ethanol was prepared by using purified water. The prepared protein sample in an amount of 20 µL and 300 µL of the immobilizing reagent solution were mixed. The thus obtained 320 µL of immobilizing reagent solution was immobilized, washed, stained, and measured an absorbance (signal intensity) at 600 nm using a densitometer in the same manner as in Example 1.
   Determination of protein by the liquid-phase method
   Pyromolex solution (Protein assay rapid kit Wako) in an amount of 1 mL was added to 20 µL of the protein sample prepared above, and then incubated at room temperature for 20 minutes to measure an absorbance at 600 nm.

### Results

The thus obtained results are summarized in Table 3 by converting to relative values, in which the result obtained by using corresponding control (the sample without containing SDS) is set as 100.

**Table 3**

| | Solid-phase method | | | Liquid-phase method | | |
|---|---|---|---|---|---|---|
| | Control | Containing 0.2% (W/V) SDS | Containing 2% (W/V) SDS | Control | Containing 0.2% (W/V) SDS | Containing 2% (W/V) SDS |
| BSA | 100 | 95 | 83 | 100 | 0 | 0 |
| Trypsin inhibitor | 100 | 106 | 101 | 100 | 0 | 0 |
| Fibrinogen | 100 | 107 | 91 | 100 | 35 | 0 |
| Hemoglobin | 100 | 119 | 104 | 100 | 0 | 0 |
| OVA | 100 | 105 | 104 | 100 | 2 | 0 |
| Cytochrome c | 100 | 120 | 128 | 100 | 2 | 0 |
| Lysozyme | 100 | 111 | 97 | 100 | 3 | 0 |
| IgG | 100 | 107 | 104 | 100 | 34 | 0 |
| Trypsin | 100 | 87 | 103 | 100 | 0 | 0 |

As is clear from Table 3, when the protein is measured by the solid-phase method of the present invention, the measurement results within ±20% to the control can be obtained in almost all proteins even in the sample containing 2% (W/V) SDS, and it is understood that detrimental effect of SDS in the protein sample on the determination of protein can be avoided.
Contrary, when the measurement is performed with the liquid-phase method using the sample containing 2% (W/V) SDS, the measurement could not be achieved at all.
From the above results, it is understood that the method for immobilizing a protein and the method for quantitative determination of protein of the present invention can be applied to all proteins. Also, the method became clear to be further more useful on the point that the quantitative determination of a protein in a sample containing a known inhibitor for determining protein, especially SDS which is widely used as a solubilizing agent for protein.

### Example 9

Effect of a surfactant in a sample on the immobilization method and a protein determination using immobilizing reagent solution of the present invention was examined.
Immobilization of protein by the solid-phase method and determination of protein
(1) Determination of protein in a sample containing BSA or IgG
   BSA samples or IgG samples (an amount of protein: 4 µg/20 µL) each containing a surfactant so as to obtain the concentrations described in Table 4 were prepared with purified water as the protein samples.
   Separately, immobilizing reagent solution containing 0.1% (WN) SDS, 2.5% (WN) TCA and 45% (VN) ethanol was prepared with purified water.
   A 20 µL of the protein sample and 300 µL of the immobilizing reagent solution were mixed to prepare the immobilizing reagent solution, and using the 320 µL obtained thereof, a protein in the immobilization sample was immobilized to a PVDF membrane in the same manner as in Example 1, washed, stained, and measured an absorbance (signal intensity) at 600 nm.
   A final concentration of each reagent in the immobilization sample is SDS 0.094% (WN), TCA 23.4% (WN) and ethanol 42.2% (VN), when the protein sample without containing SDS is used. With regard to the final concentration of each reagent in the case using a protein sample containing SDS, the final concentrations of TCA and ethanol are the same as in the case using the protein sample without containing SDS. Final concentrations of SDS are: 0.16% (WN) in the case using a protein sample containing 1% SDS; 0.21 % (WN) in the case using a protein sample containing 2% SDS; and 0.34% (WN) in the case using a protein sample containing 4% SDS.

(2) Determination of protein in a sample containing OVA
   OVA samples (an amount of protein: 5µg/20 µL) each containing a surfactant so as to obtain the concentrations described in Table 4 were prepared as protein samples.
   Separately, immobilizing reagent solution containing 0.2% (WN) SDS, 2.5% (WN) TCA and 45% (VN) ethanol was prepared with purified water, and a immobilization sample was prepared in the similar manner as in the above (1), then the protein in the immobilization sample was immobilized to a PVDF membrane in the same manner as in example 1, washed, stained, and measured an absorbance (signal intensity) at 600 nm.
   A final concentration of each reagent in the immobilization sample is SDS 0.19% (WN), TCA 23.4% (WN) and ethanol 42.2% (VN), when the protein sample without containing SDS is used. Further, a final concentration of each reagent in the case using the protein sample containing 2% SDS is SDS 0.31% (WN), TCA 23.4% (WN) and ethanol 42.2% (VN).
   Further, immobilization and determination were performed in the same manner as above by using a BSA sample, an IgG sample and an OVA sample prepared by the same manner as above except for without containing surfactant (inhibitor), and the results were used as a control.
   Determination of protein by the liquid-phase method
   BSA samples (10 µg/20 µL) each containing a surfactant was prepared so as to obtain the concentrations described in Table 4, and determination was performed in the same manner as in Example 6 using 1 mL of Pyromolex solution.
   Further, determination was performed by the liquid-phase method in the same manner using the BSA sample prepared by the same manner as above except for without containing surfactant (inhibitor), and the results were used as control.

### Results

Each determination procedure was repeated three times, and a mean value of the obtained absorbance was calculated. A mean value obtained by using the control is set as 100. A relative value (%), i.e. a mean value of the absorbance obtained by using a sample containing the surfactant to the absorbance of the control, is calculated and shown in Table 4 with the coefficient variation (CV).
In Table 4, concentrations of surfactants (inhibitors) show concentrations in a protein sample. In the data on the liquid-phase method, values in the left side indicate the concentration of a surfactant, and a value in the right side indicates a relative value of a mean measurement value in the case using a sample containing a surfactant to the control (%) ±CV,
The data shown in Table 4 indicate values for the maximum allowable concentration of a surfactant, i.e. the data in the case using a surfactant as an additive when a mean value is in a range of ±20% of the control.

**Table 4**

| | | **Solid-phase method** | | | **Liquid-phase method** |
|---|---|---|---|---|---|
| **Surfactant** | **Concentration** | BSA (4µg) mean(%) ± CV | IgG (4µg) mean(%) ± CV | OVA (5µg) mean(%) ± CV | BSA (10µg) mean(%) ± CV |
| SDS | 1% (w/v) | 87 ±7.1 | | | (0.01% (w/v), 92±6.0) |
| | 2% (w/v) | 82 ±6.2 | 100 ±0.9 | 117 ±2.2 | |
| | 4% (w/v) | | 103 ±4.3 | | |
| SLS | 2% (w/v) | | 101 ±1.9 | | (0.1% (w/v),109±7.3) |
| | 3% (w/v) | 94 ±3,7 | | | |
| Triton X- 100 | 1% (w/v) | | 112 ±0.2 | | (0.1% (w/v), 107±3.9, |
| | 2% (w/v) | 98 ±1.9 | 115 ±1.5 | 113 ±3.0 | |
| NP-40 | 1% (w/v) | 96 ±4.5 | 115 ±3.0 | | (0.1% (w/v),116±4.7) |
| | 2% (w/v) | 90 ±1.2 | | 115 ±2.4 | |
| Tween 20 | 0.05% (w/v) | | 110 ±4.4 | | |
| | 0.1% (w/v) | 97 ±4.5 | 116 ±2.8 | | 115 ±1.0 |
| | 0.2% (w/v) | 89 ±2.6 | | 109 ±1.6 | |
| Tween 80 | 0.05% (w/v) | 105 ±2.7 | | | |
| | 0.1% (w/v) | 82 ±2.0 | 112 ±0.6 | 111 ±0.7 | 112 ±1.4 |
| Briji 35 | 1% (w/v) | | 107 ±3.4 | | (0.1% (w/v), 108±3.2) |
| | 2% (w/v) | 93 ±3.9 | | 112 ±2.9 | |
| CHAPS | 1% (w/v) | 96 ±2.6 | 111 ±2.2 | | (0.1% (w/v), 104±0.0) |
| | 2% (w/v) | 91 ±1.3 | 116 ±1.3 | 106 ±0.9 | |
| CTAB | 0.05% (w/v) | | | 112 ±0.2 | |
| | 0.1% (w/v) | | | 123 ±1.0 | |

SLS: sodium N-lauroylsarcosinate
Triton X-100 (Rohm and Haas inc., Trade name): polyoxyethylene (10) octylphenyl ether
NP-40 (Nippon Emulsion Co. Ltd., Trade name): Polyoxyethylene (9) octylphenyl ether
Tween 20 (Kao Corp., Trade name): Poly(oxyethylene) sorbitan monolaulate
Tween 80 (Kao Corp., Trade name): Poly(oxyethylene) sorbitan monooleate
Brij 35 (ICI Plc, Trade name): Polyoxyethylene lauryl ether
CHAPS: 3-[(3-Cholamidopropyl)dimethylammonio]propanesulfonic acid
CTAB: Cetyl trimethyl ammonium bromide
As is clear from Table 4, when a protein is immobilized and determined by the method of the present invention, it is understood that determination of the protein can be achieved without receiving a detrimental effect, even a surfactant generally used is exist in high concentration when a protein sample is prepared. In particular, in comparison with the liquid-phase method, it is understood that the measurement by the solid-phase method can be made even when ten-fold or more concentration of a surfactant coexists in a protein sample as an additive.

Accordingly, it is understood that the immobilization method of a protein of the present invention can solve the problem caused by a surfactant that is widely used as a conventional additive, that is, the problem of inhibition of the quantitative determination of a protein.

### Example 10. Immunoblotting

### Preparation of sample and reagent solution

(1) Protein sample
   Mouse IgG (Wako Pure Chemical Industries Ltd.) was weighed and dissolved in purified water to prepare 0 to 200 µg/mL of solution, and used as the protein sample.
(2) Immobilizing reagent solution
   Immobilizing reagent solution containing 0.2% (W/V) SDS, 2.5% (W/V) TCA and 45% (V/V) ethanol was prepared by using purified water.
(3) Immobilization sample
   Each protein sample in an amount of 20 µL having the above concentration prepared above and 300 µL of the immobilizing reagent solution were mixed (final concentration of SDS 0.19% (WN), TCA 2.34% (WN) and ethanol 42.2% (V/V)) to prepare a solution for use as the immobilization samples.
(4) Blocking solution
   Block Ace (Snow Brand Milk Products Co. Ltd.) diluted with PBS (pH 7.4) so as to be final concentration 25% was used.
(5) Antibody solution
   Antibody solution for detecting luminescence: Horseradish peroxidase labeled anti-mouse IgG antibody (Amersham Biosciences) diluted to 1/10000 with the blocking solution was used.

Antibody solution for detecting coloring: Alkaline phosphatase labeled anti-mouse IgG antibody (Wako Pure Chemical Industries Ltd.) diluted to 1/1000 with a blocking solution was used.
(6) Washing solution
   Tween 20 diluted with PBS (pH 7.4) to final concentration 0.05% was used.
(7) Reagent for detection
   For the detection of luminescence: ECL Plus Western Blotting Starter Kit
(Amersham Biosciences)
For the detection of coloring: 0.033% of nitro blue tetrazolium (NBT, Wako Pure Chemical Industries Ltd.), 0.0165% of 5-bromo-4-chloro-3-indolyl phosphate (BCIP, Wako Pure Chemical Industries Ltd.) / 100mM Tris-HCl, pH 9.5 (containing 100 mM NaCl and 5 mM MgCl₂)

### Immobilization and determination of protein

The immobilization sample prepared above was applied on a PVDF membrane, suction filtered, and applied with 300 µL of PBS (pH 7.4) and suction filtered as the same manner as in Example 1 The PVDF membrane was taken out, immersed into a blocking solution, and incubated at room temperature for 1 hour with rotating (blocking procedure). Subsequently, the membrane was immersed into an antibody solution for detection of luminescence or the antibody solution for detection of coloring, and incubated at room temperature for 1 hour with rotation (antibody reaction). The remembrance after antibody reaction was washed with the washing solution for five times. The obtained membrane was immersed into the reagent for the detection of luminescence or the solution for the detection of coloring, and reaction for determination is performed. Detection of luminescence was conducted as follows. After the PVDF membrane was treated by luminescent detection, the membrane was exposed to a X-ray film (Amersham Biosciences) for detection.

### Results

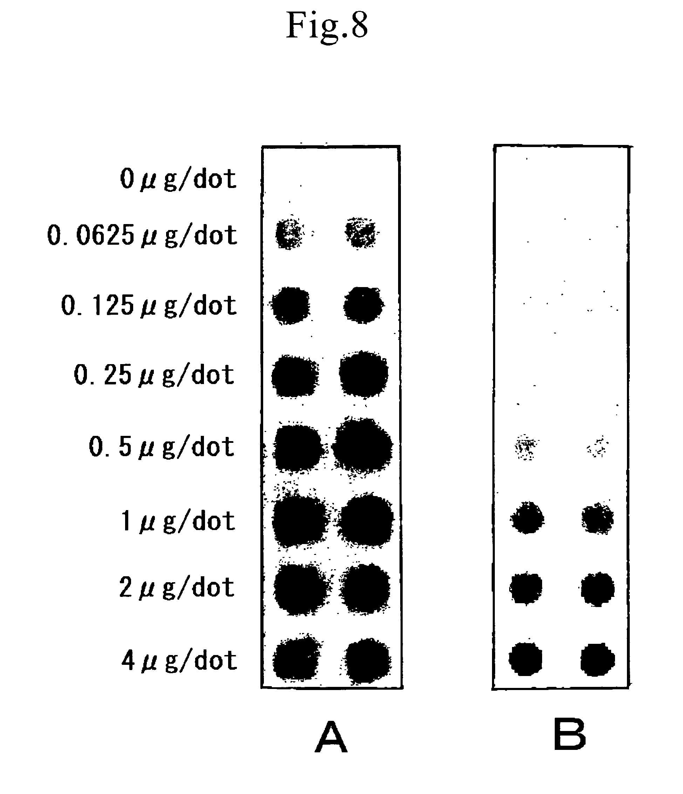

The results are shown in Fig. 8. In Fig. 8, A shows the results obtained by performing an immunodetection of the protein sample immobilized to the PVDF membrane by luminescent reaction, and exposing the membrane to an X-ray film in order to detect. B shows the results obtained by performing an immunodetection of the protein sample immobilized to the PVDF membrane by coloring reaction in order to detect. Each dot shows the results of detection after immobilizing protein samples with each amount of the protein.
As is clear from Fig. 8, mouse IgG immobilized to the membrane could be detected in any case of luminescence detection and coloring detection by immunoblotting. Detection limit in the luminescent detection was 0.0625 µg and the detection limit in the coloring detection was 0.5 µg. Consequently, it is understood that a protein immobilized by the immobilization method of the present invention can be used for immunological detection by the immunoblotting.

### Example 11

### Preparation of sample to be tested containing abnormal PrP

(1) Preparation of reagent solutions: Following reagent solutions were prepared.
   (i) Homogenizing Solution
      A 50 mM Tris-HCl, pH 7.4 (containing 150 mM NaCl, 1 mM KCl, 1 mM EDTA, 1% (WN) sodium deoxycholate and 1% (WN) Triton X-100) was used.
   (ii) Protease solution
      Proteinase K (derived from Tritirachium album, Wako Pure Chemical Industries Ltd.) in an amount of 200 µg was dissolved in 1.0 mL of 10 mM Tris-HCl pH 7.4 (containing 150 mM NaCl, 1 mM KCl, 1 mM EDTA, 20% (WN) SDS and 10% (WN) Triton X-100).
   (iii) Protein precipitation agent
      Mixed solvent of 2-butanol / methanol (5 : 1, W/W).
   (iv) Precipitate dissolving solution
      A 50 mM Tris-HCl pH 7.4 (containing 150 mM NaCl, 1 mM KCl, 1 mM EDTA, 0.005% (WN) BSA and 2% (WN) SDS) was used.

(2) Preparation of sample to be tested containing abnormal PrP
   A small tissue pieces of a bovine cerebellum infected with BSE (repeated freeze-thaw) in an amount of 320 mg (wet weight) was homogenized in 1.3 mL of the homogenizing solution using a multi-beads shocker (Yasui Kikai Co.) and centrifuged at 20°C and 5000 × g, for 5 minutes to obtain a supernatant.
   Protease solution in an amount of 500 µL was added to the thus obtained supernatant, mixed well and reacted at 37°C for 10 minutes, then 500 µL of protein precipitation agent was added and mixed.
   Subsequently, the solution was centrifuged at 20000 × g for 5 minutes and the supernatant was withdrawn to obtain the precipitate.
   After dissolving the obtained precipitate by adding 60 µL of the precipitate dissolving solution, the solution was treated at 100°C for 5 minutes to inactivate proteinase K and inactivate pathogenicity of abnormal PrP.
   The thus obtained solution was used as a sample to be tested containing abnormal PrP.

### Immobilization and detection of abnormal PrP

(1) Preparation of reagent solution
   (i) Immobilizing reagent solution 1
      Immobilizing reagent solution 1 containing 0.1% (WN) SDS, 2.5% (WN) TCA and 45% (VN) ethanol was prepared by using purified water.
   (ii) Immobilizing reagent solution 2
      Immobilizing reagent solution 2 containing 0.1% (WN) Tween 20, 2.5% (WN) TCA and 45% (VN) ethanol was prepared by using purified water.
   (iii) Sample for immobilizing abnormal PrP
      A mixture of 20 µL of the sample to be tested containing abnormal PrP prepared above and 200 µL of the immobilizing reagent solution 1 was prepared (final concentration of SDS 0.27% (WN), TCA 2.3% (WN) and ethanol 41% (V/V)) for use as a sample for immobilizing abnormal PrP.
   (iv) Washing solution
      Tween 20 diluted with PBS (pH 7.4, containing 0.02% (VN) Suraoff, (Japan EnviroChemicals, Ltd., Trade name)) to final concentration 0.05% was used.
   (v) Antibody solution
      Horse radish peroxidase labeled anti-BSE monoclonal antibody (National Institute of Animal Health, National Agriculture and Bio-oriented Research Organization) diluted to 1/500 with PBS (pH 7.4, containing 20% (WN) glycerol and 0.1% (WN) BSA) was used.
   (vi) Coloring reagent solution
      TMB solution (Wako Pure Chemical Industries Ltd. for micro well) was used.
   (vii) Stop solution
      0.1 M HCl aqueous solution was used as the stop solution.

(2) Immobilization and detection of abnormal PrP
   The sample for immobilizing abnormal PrP prepared above was applied to wells of multiplate (Millipore Corp.), to which the PVDF membrane was set, allowed to stand for 5 minutes and suction filtered. Subsequently, 100 µL of the immobilizing reagent solution 2 was applied, and suction filtered in the same manner as above to wash the PVDF membrane. This washing procedure was performed again.
   Thereafter, 50µL of the antibody solution was applied to the wells and reacted for 20 minutes. After the reaction, the procedures of applying 300 µL of washing solution and suction filtration were repeated five times to wash the PVDF membrane.
   Then 50 µL of the coloring reagent solution was applied and reacted at room temperature for 30 minutes in the dark room.
   After the reaction, the reaction mixture in the wells of the multiplate was transferred to wells of 96 well ELISA plate by suction filtration. Thereafter, 50 µL of the coloring reagent solution was applied again to the each well of the multiplate on which abnormal PrP was immobilized, and the coloring reagent solution was transferred to the corresponding same wells of 96 well ELISA plate by suction filtration, and the wells were washed concurrently with the reaction mixture. Then 100 µL of the stop solution was applied to each well of the 96 well ELISA plate to terminate the reaction.
   Absorbance (dominant wavelength 450 nm, sub-wavelength 620 nm) of the reaction mixture was measured by using microplate reader (Molecular Devices Corp.) within 30 minutes after termination of the reaction.
   Immobilization of negative control (50 mM Tris-HCl containing 0.01% (W/V) BSA and 0.02% Suraoff, pH 7.4) and positive control (containing 50 mM Tris-HCl, pH 7.4, recombinant PrP 50 µg/mL (rPrP, supplied from Hiroshima University), 150 mM NaCl, 1 mM KCl, 1 mM EDTA, 0.01% (W/V) BSA and 2% (W/V) SDS), and measurement of absorbance were performed. The results are shown in Table 5.

### Comparative Example 1 (Conventional ELISA method)

Abnormal PrP was detected by using Plateria (Trade Mark) BSE kit (Japan Bio-Rad Laboratories Inc.) as follows.
Preparation of sample to be tested containing abnormal PrP
A small tissue pieces of the bovine cerebellum infected with BSE (repeated
freeze-thaw), which was the same as that used in Example 1 in an amount of 350 mg (wet weight), was homogenized in 1.4 mL of the homogenizing solution (glucose 50 mg/mL) using a multi-beads shocker.
A solution prepared by diluting protease K (derived from Tritirachium album) attached to the kit with Reagent A solution (urea 0.12 g/mL) in an amount of 500 µL was added to the thus obtained homogenate, and reacted at 37°C for 10 minutes.
Subsequently, 500 µL of Reagent B solution (3-butanol) was added, mixed well, and centrifuged at 20000 × g for 5 minutes to obtain a precipitate.
Reagent C 1 solution (urea 0.36 g/mL) was added to and dissolved the precipitate, treated at 100°C for 5 minutes to inactivate proteinase K and pathogenicity of abnormal PrP.
The thus obtained solution was used as a sample to be tested containing abnormal PrP.

### Detection of abnormal PrP

The sample to be tested containing abnormal PrP prepared above in an amount of 17 µL was dissolved in 83 µL of a dilution solution, and was added dropwise into a 96 well ELISA plastic plate bound with anti-human PrP mouse monoclonal antibody, then allowed to stand at 37°C for 75 minutes. Subsequently, a procedure of washing the well with 350 µL of washing solution was repeated six times.
POD labeled anti-human PrP mouse monoclonal antibody solution in an amount of 100 µL was added to each well and reacted at 4°C for 60 minutes.
Each well was washed with washing solution (Tris buffer) for ten times. After washing, 100 µL of the substrate coloring reagent solution (TMB solution) was applied to each well, and reacted for 30 minutes. After the reaction, 100 µL of the stop solution (0.5 M sulfuric acid) was added to each well to terminate the reaction.
Absorbance was measured with the dominant wavelength at 450 nm and sub-wavelength at 620 nm by using microplate reader (Molecular Devices Corp.).
Separately, immobilization and measurement of absorbance were performed using the negative control (PBS containing 0.1% (W/V) BSA) attached to the kit and the positive control (synthesized peptide of human prion protein) attached to the kit.
The results are shown in Table 5 together.

**Table 5**

| | Absorbance | | |
|---|---|---|---|
| | Negative Control | Positive Control | Sample to be tested |
| Example 11 | 0.082 | 3.258 | 3.099 |
| Comparative Example 1 | 0.072 | 1.500 | 0.385 |

As is clear from above, a process from the immobilization to the detection could be performed within 60 minutes in Example 11, whereas a time of 75 minutes was required only for the step of protein immobilization in Comparative Example 1. Consequently, it is understood that the method of the present invention can be performed a determination of BSE infection more rapidly.
Further, as is clear from Table 5, it is understood that the absorbance of BSE specimen is obviously lower than the positive control in Comparative Example 1 when BSE specimen (sample to be tested) was used, and therefore, a highly reliable determination of BSE can not be performed with the samples of BSE infected bovine cerebellum with repeated freezing and thawing. Contrary, the absorbance equivalent to the positive control could be measured in Example 11. Consequently, determination of BSE can be achieved even in any condition of the specimen.

### Example 12. Setting cutoff value

### Preparation of sample to be tested

(1) Preparation of reagent solution: Following reagent solutions were prepared.
   (i) Homogenizing solution
      A 50 mM Tris-HCl pH 7.4 (containing 150 mM NaCl, 1 mM KCl, 1 mM EDTA, 1% (W/V) sodium deoxycholate and 1% (W/V) Sunlight SL-50 (nonionic surfactant, Sunlight K.K., Trade name)) was used.
   (ii) Protease solution
      Proteinase K (derived from Tritirachium album, Wako Pure Chemical Industries Ltd.) in an amount of 100 µg was dissolved in 500 µL of 10 mM Tris-HCl pH 7.4 (containing 150 mM NaCl, 1 mM KCl, 1 mM EDTA, 20% (W/V) SLS and 10% (W/V) Sunlight SL-50) (final concentration of proteinase K: 200 µg/mL) was used.
   (iii) Protein precipitation agent
      Mixed solvent of 2-butanol / methanol (5 : 1, W/W)
   (iv) Precipitate dissolving solution
      50 mM Tris-HCl pH 7.4 (containing 150 mM NaCl, 1 mM KCl, 1 mM EDTA, 0.005% (W/V) BSA and 2% (W/V) SDS) was used.

(2) Preparation of sample to be tested
   Small tissue pieces of the obex of medulla oblongata of 48 bovines which had been confirmed not to be infected with BSE (negative specimen, 48 specimens) in an amount of 320 to 350 mg (wet weight) each were collected, homogenized at 2000 rpm for 30 seconds using a multi beads shocker (Yasui Kikai Co.) in 1.3 mL of the homogenizing solution, and centrifuged at room temperature and 5000 × g, for 5 minutes to obtain supernatant.
   Protease solution in an amount of 500 µL (Proteinase K: 100 µg) was added to 500 µL of the thus obtained supernatant, and mixed well. After reacting at 37°C for 10 minutes, 500 µL of the protein precipitation agent was added and mixed.
   The mixture was centrifuged at 20000 × g for 5 minutes, and the supernatant was withdrawn to obtain a precipitate.
   After dissolving the obtained precipitate by adding 60 µL of the precipitation dissolving solution, the solution was treated at 100°C for 5 minutes to inactivate proteinase K and pathogenicity of abnormal PrP.
   The thus obtained solution was used as a sample to be tested.

### Immobilization and detection of PrP

(1) Preparation of reagent solution
   (i) Immobilizing reagent solution 1
      Immobilizing reagent solution 1 containing 0.1% (WN) SDS, 2.5% (WN) TCA and 45% (VN) ethanol was prepared by using purified water.
   (ii) Immobilizing reagent solution 2
      Immobilizing reagent solution 2 containing 0.1% (WN) Tween 20, 2.5% (WN) TCA and 45% (VN) ethanol was prepared by using purified water.
   (iii) Sample for immobilizing PrP
      A mixture of 60 µL of the sample to be tested prepared above were collected in a microtube, and 600 µL of the immobilizing reagent solution 1 was added (final concentration of SDS 0.27% (WN), TCA 2.3% (WN) and ethanol 41% (V/V)) to prepare a sample for immobilizing PrP.
   (iv) Washing solution
      Tween 20 diluted with PBS (pH 7.4, containing 0.02% (V/V) Suraoff, (Japan EnviroChemicals, Ltd., Trade name)) to final concentration 0.05% was used.
   (v) Antibody solution
      Horse radish peroxidase labeled anti-BSE monoclonal antibody (National Institute of Animal Health, National Agriculture and Bio-oriented Research Organization) diluted to 1/500 with PBS (pH 7.4, containing 20% (WN) glycerol and 0.1% (WN) BSA) was used.
   (vi) Coloring reagent solution
      TMB solution (Wako Pure Chemical Industries Ltd. for micro well) was used.
   (vii) Stop solution
      0.1 M HCl aqueous solution was used as a stop solution.

(2) Immobilization and detection of PrP
   In each of the sample for immobilizing PrP prepared above, 600 µL in each sample was applied to three wells (triplicate of each sample, i.e. 200 µL/well each) of a multiplate to which a PVDF membrane was set.
   The multiplate was allowed to stand at room temperature for 5 minutes and the solution in each well was suction filtered. Subsequently, 100 µL each of the immobilizing reagent solution 2 was applied to each well, and suction filtered in the same manner as above. Further, 300 µL of the washing solution was applied to each well, and suction filtered in the same manner as above to wash then the PVDF membrane of each well. This washing procedure was repeated three times. Thereafter, 50µL of the antibody solution was applied to each well and reacted at room temperature for 20 minutes. After the reaction, the procedure of applying 300 µL of washing solution in each well and suction filtration was repeated five times to wash the PVDF membrane.
   The coloring reagent solution in an amount of 50 µL was applied to each well and reacted at room temperature for 30 minutes in the dark place.
   After the reaction, the reaction mixture in the wells of multiplate was transferred to wells of 96 well ELISA plate by suction filtration. Thereafter, 50 µL each of the coloring reagent solution was applied again to the each well of the multiplate on which PrP was immobilized, and the solution was transferred to the corresponding same wells of the 96 well ELISA plate by suction filtration, and the wells were washed concurrently with the reaction mixture. Then 100 µL of the stop solution was applied to each well of 96 well ELISA plate to terminate the reaction.
   Absorbance (dominant wavelength at 450 nm, sub-wavelength at 620 nm) of the reaction mixture was measured by using microplate reader (Molecular Devices Corp.) within 30 minutes after termination of the reaction. Mean value, SD and CV(%) of the absorbance of the triplicate measured in each specimen are shown in Table 6.
   Separately, immobilization of negative control (50 mM Tris-HCl containing 0.01% (W/V) BSA and 0.02% Suraoff, pH 7.4) and positive control (containing 50 mM Tris-HCl, pH 7.4, recombinant PrP 50 µg/mL (rPrP, supplied from Hiroshima University), 150 mM NaCl, 1 mM KCl, 1 mM EDTA, 0.01% (W/V) BSA and 2% (W/V) SDS), and the measurement of absorbance were performed. The negative control was prepared for 3 specimens and the positive control was prepared for 2 specimens, and the measurement was performed in triplicate for each specimen. The mean value of absorbance obtained for each specimen is shown in Table 7.

### Comparative Example 2 (Conventional ELISA method)

PrP was detected by using Plateria (Trade Mark) BSE kit (Japan Bio-Rad Laboratories Inc.) in the same manner as in Comparative Example 1 as follows. Preparation of sample to be tested
A small pieces of negative specimen of 48 specimens, which were the same as that used in Example 12, in an amount of 320 to 350 mg (wet weight) were collected, and each piece is homogenized in 1.4 mL of homogenizing solution (glucose 50 mg/mL) by using a multi-beads shocker.
A solution prepared by diluting protease K (derived from Tritirachium album) attached to the kit with Reagent A solution (urea 0.12 g/mL) in an amount of 500 µL of was added to 500 µL of the obtained homogenate, and reacted at 37°C for 10 minutes.
Subsequently, 500 µL of Reagent B solution (3-butanol) was added, mixed well, and centrifuged at 20000 × g for 5 minutes to obtain a precipitate.
Reagent C1 solution (urea 0.36 g/mL) was added to and dissolved the precipitate, treated at 100°C for 5 minutes to inactivate proteinase K and pathogenicity of abnormal PrP.
The thus obtained solution was used as a sample to be tested.

### Detection of PrP

The sample to be tested in an amount of 17 µL prepared above was dissolved in 83 µL of the dilution solution, and the solution was added dropwise into a 96 well ELISA plastic plate bound with anti-human PrP mouse monoclonal antibody, then allowed to stand at 37°C for 75 minutes. Subsequently, a procedure of washing the well with 350 µL of washing solution was repeated six times.
POD labeled anti-human PrP mouse monoclonal antibody solution in an amount of 100 µL was added to each well and reacted at 4°C for 60 minutes.
Each well was washed ten times with a washing solution (Tris buffer). After washing, 100 µL of a substrate coloring reagent solution (TMB solution) was applied to each well, and reacted for 30 minutes. After the reaction, 100 µL of the stop solution (0.5 M sulfuric acid) was added to each well to terminate the reaction.
Absorbance was measured with the dominant wavelength at 450 nm and sub-wavelength at 620 nm by using a microplate reader (Molecular Devices Corp.). The results are shown in Table 6 together.
Separately, immobilization and measurement of absorbance were performed using two specimens of the negative control (PBS containing 0.1% (W/V) BSA) attached to the kit and two specimens of the positive control (synthesized peptide of human prion protein) attached to the kit.
The results are shown in Table 7 together.

**Table 6**

| Sample No. | Example 12 | | | Comparative Example 2 |
|---|---|---|---|---|
| | mean | SD | CV% | |
| No.1 | 0.029 | 0.025 | 87% | 0.073 |
| No.2 | 0.040 | 0.006 | 15% | 0.110 |
| No.3 | 0.025 | 0.008 | 33% | 0.065 |
| No.4 | 0.032 | 0.015 | 45% | 0.070 |
| No.5 | 0.036 | 0.032 | 87% | 0.054 |
| No.6 | 0.065 | 0.014 | 22% | 0.046 |
| No.7 | 0.055 | 0.005 | 8% | 0.079 |
| No.8 | 0.032 | 0.028 | 89% | 0.048 |
| No.9 | 0.040 | 0.017 | 41% | 0.054 |
| No.10 | 0.050 | 0.002 | 5% | 0.035 |
| No.11 | 0.066 | 0.017 | 25% | 0.040 |
| No.12 | 0.065 | 0.007 | 10% | 0.043 |
| No.13 | 0.053 | 0.016 | 30% | 0.026 |
| No.14 | 0.060 | 0.020 | 33% | 0.026 |
| No.15 | 0.054 | 0.010 | 18% | 0.036 |
| No.16 | 0.061 | 0.026 | 42% | 0.026 |
| No.17 | 0.056 | 0.021 | 37% | 0.056 |
| No.18 | 0.041 | 0.001 | 2% | 0.033 |
| No.19 | 0.044 | 0.005 | 12% | 0.038 |
| No.20 | 0.042 | 0.006 | 14% | 0.025 |
| No.21 | 0.058 | 0.040 | 69% | 0.034 |
| No.22 | 0.066 | 0.007 | 11% | 0.027 |
| No.23 | 0.048 | 0.002 | 5% | 0.027 |
| No.24 | 0.051 | 0.023 | 46% | 0.037 |
| No.25 | 0.019 | 0.020 | 104% | 0.056 |
| No.26 | 0.040 | 0.005 | 11% | 0.046 |
| No.27 | 0.024 | 0.009 | 35% | 0.055 |
| No.28 | 0.034 | 0.019 | 56% | 0.036 |
| No.29 | 0.041 | 0.040 | 96% | 0.050 |
| No.30 | 0.068 | 0.007 | 10% | 0.050 |
| No.31 | 0.079 | 0.010 | 12% | 0.058 |
| No.32 | 0.034 | 0.028 | 84% | 0.053 |
| No.33 | 0.064 | 0.053 | 83% | 0.137 |
| No.34 | 0.054 | 0.003 | 5% | 0.064 |
| No.35 | 0.071 | 0.017 | 24% | 0.064 |
| No.36 | 0.093 | 0.027 | 29% | 0.066 |
| No.37 | 0.055 | 0.016 | 30% | 0.066 |
| No.38 | 0.062 | 0.020 | 32% | 0.077 |
| No.39 | 0.057 | 0.010 | 17% | 0.081 |
| No.40 | 0.063 | 0.026 | 41% | 0.051 |
| No.41 | 0.088 | 0.002 | 2% | 0.059 |
| No.42 | 0.044 | 0.002 | 4% | 0.054 |
| No.43 | 0.046 | 0.006 | 12% | 0.066 |
| No.44 | 0.044 | 0.008 | 17% | 0.032 |
| No.45 | 0.074 | 0.047 | 64% | 0.033 |
| No.46 | 0.083 | 0.010 | 11% | 0.028 |
| No.47 | 0.052 | 0.002 | 3% | 0.040 |
| No.48 | 0.052 | 0.025 | 49% | 0.051 |

**Table 7**

| | Sample No. | Example 12 | Comparative Example 2 |
|---|---|---|---|
| Negative Control | 1 | 0.049 | 0.014 |
| | 2 | 0.065 | 0.016 |
| | 3 | 0.075 | 0.012 |
| Positive Control | 1 | 1.262 | 1.989 |
| | 2 | 1.323 | 2.07 |

According to the instruction of BSE kit used in Comparative Example 2, when the absorbance in the negative control is 0.15 or less, and besides the absorbance in the positive control is 1.0 or more, the detection system is judged to be reliable. As is clear from above Table 7, the absorbance of the negative control was 0.15 or less and an absorbance of the positive control was 1.0 or more in the both case that the measurement was performed by the detection method for PrP of the present invention (Example 12) and the measurement was performed by the detection method for PrP using the conventional BSE kit (Comparative Example 2).
From this fact, the detection system in the detection method for PrP of the present invention can be judged to be reliable as the same as in the conventional detection method by ELISA.

A mean value of the absorbance of the negative specimens was calculated according to the obtained results of Table 6 above, and found to be: mean value was 0.052 ± 0.017 and CV value (%) was 32%.
Contrary, the mean absorbance was 0.052 ± 0.022 and CV value (%) was 42% in the negative specimen (48 specimens), when the detection of PrP was performed in the case using the BSE kit by means of the conventional ELISA. Namely, it is understood that the detection method for PrP of the present invention shows a less variation than the method using the conventional BSE kit in the measured values for each negative specimen.

Further, a cutoff value for determination of BSE infection was defined. The brain is mainly used as specimen in the determination of BSE in PrP, but substances other than PrP of the brain tissues may cause to generate a nonspecific reaction to anti-PrP antibody. Further, considering a possibility of causing an error of measurement in the measurement procedure, in the case of Example 12, a value of 0.203, which was obtained by adding a mean value of absorbance for the negative specimen performed by the method in Example 12 obtained in above Table 7 (0.63) to a mean value of absorbance for the negative specimen, i.e. (0.052) + 5SD(5 × 0.017) ≒ 0.14, was tentatively set as a cutoff value for determining BSE infection. In the case of Comparative Example 2, according to the instruction attached to the kit, a value of 0.224, which was obtained by adding 0.21 as described in the instruction to a mean value of absorbance for the negative specimen performed by the method in Comparative Example 2 obtained in Table 7 above (0.014), was tentatively set as a cutoff value for determining BSE infection.

### Example 13. Determination of BSE

### Preparation of sample to be tested

(1) Preparation of reagent solution
   Each reagent solution was prepared according to the method in the same manner as in Example 12.
(2) Preparation of sample to be tested
   A small tissue pieces of the bovine cerebellum infected with BSE (positive specimen, 10 specimens) in an amount of 320 to 350 mg (wet weight) was collected and homogenized in 1.3 mL of the homogenizing solution by using a multi-beads shocker (Yasui Kikai Co.) at 2000 rpm for 30 seconds, and centrifuged at room temperature and 5000 × g, for 5 minutes to obtain a supernatant.
   A protease solution (proteinase K 100 µg) in an amount of 500 µL was added to 500 µL of the thus obtained supernatant, mixed well and reacted at 37°C for 10 minutes, then 500 µL of protein precipitation agent was added and mixed.
   Subsequently, the solution was centrifuged at 20000 × g for 5 minutes and the supernatant was withdrawn to obtain a precipitate.
   After dissolving the obtained precipitate by adding 60 µL of the precipitate dissolving solution, the solution was treated at 100°C for 5 minutes to inactivate proteinase K and pathogenicity of abnormal PrP.
   The thus obtained solution was diluted two-fold or ten-fold with the same precipitation dissolving solution as in Example 12 to prepare two samples to be tested.

### Immobilization and detection of PrP

(1) Preparation of reagent solution
   The same solution as in Example 12 was prepared.
   Incidentally, the samples for immobilizing PrP were prepared by the same manner as in Example 12 by using the sample to be tested by diluting two-fold or ten-fold with the homogenizing solution prepared above.

(2) Immobilization and detection of PrP
   In each of the sample for immobilizing PrP prepared above, 600 µL in each sample, was applied to 3 wells (triplicate of each sample, i.e. 200 µL/well each) of a multiplate to which PVDF membrane was set.
   The multiplate was allowed to stand at room temperature for 5 minutes and the solution in each well was suction filtered. Subsequently, 100 µL each of the immobilizing reagent solution 2 was applied to each well, and suction filtered in the same manner as above. Further, 300 µL of the washing solution was applied to each well, and suction filtered in the same manner as above to wash the PVDF membrane of each well. This washing procedure was repeated three times. Thereafter, 50µL of the antibody solution was applied to each well and reacted at room temperature for 20 minutes. After the reaction, the procedure of applying 300 µL of washing solution in each well and suction filtration was repeated five times to wash the PVDF membrane.
   The coloring reagent solution in an amount of 50 µL was applied to each well and reacted at room temperature for 30 minutes in the dark place.
   After the reaction, the reaction mixture in the wells of multiplate was transferred to wells of 96 well ELISA plate by suction filtration. Thereafter, 50 µL each of the coloring reagent solution was applied again to the each well of the multiplate on which PrP was immobilized, and the solution was transferred to the corresponding same wells of 96 well ELISA plate by suction filtration, and the wells were washed concurrently with the reaction mixture. Then 100 µL of the stop solution was applied to each well of the 96 well ELISA plate to terminate the reaction.
   Absorbance (dominant wavelength at 450 nm, sub-wavelength at 620 nm) of the reaction mixture was measured by using microplate reader (Molecular Devices Corp.) within 30 minutes after termination of the reaction. Mean value, SD and CV(%) of the absorbance of the triplicate measured in each specimen are shown in Table 8-1 and Table 8-2. Table 8-1 shows the results of the case using the sample to be tested of two-fold dilution with the homogenizing solution and Table 8-2 shows the results of the case using the sample to be tested of ten-fold dilution with the homogenizing solution.
   Separately, immobilization of negative control (50 mM Tris-HCl containing 0.01% (W/V) BSA and 0.02% Suraoff, pH 7.4) and positive control (containing 50 mM Tris-HCl, pH 7.4, recombinant PrP 50 µg/mL (rPrP, supplied from Hiroshima University), 150 mM NaCl, 1 mM KCl, 1 mM EDTA, 0.01% (W/V) BSA and 2% (W/V) SDS), and the measurement of absorbance were performed. Incidentally, the negative control and the positive control were prepared for three specimens, respectively, and triplicate measurements were performed for each specimen. The mean values of the absorbance obtained for each specimen are shown in Table 9.

### Comparative Example 3 (Conventional ELISA method)

PrP was detected by using Plateria (Trade Mark) BSE kit (Japan Bio-Rad Laboratories Inc.) in the same manner as in Comparative Example 1 as follows. Preparation of sample to be tested.
The positive specimen of 10 specimens, which were the same as that used in Example 13, in an amount of 320 to 350 mg (wet weight) each was collected and homogenized in 1.4 mL of homogenizing solution (glucose 50 mg/mL) by using a multi-beads shocker.
A solution prepared by diluting protease K (derived from Tritirachium album) attached to the kit with Reagent A solution (urea 0.12 g/mL) in an amount of 500 µL was added to 500 µL of the thus obtained homogenate, and reacted at 37°C for 10 minutes.
Subsequently, 500 µL of Reagent B solution (3-butanol) was added, mixed well, and centrifuged at 20000 × g for 5 minutes to obtain a precipitate.
Reagent Cl solution (urea 0.36 g/mL) was added to and dissolved the precipitate, treated at 100°C for 5 minutes to inactivate proteinase K and pathogenicity of abnormal PrP.
Two solutions were prepared by diluting the thus obtained solution ten-fold with Reagent Cl solution (urea 0.36 g/mL) and not diluting, and used it as a samples to be tested.

### Detection of PrP

The sample to be tested in an amount of 17 µL prepared above was treated for detection of PrP by the same manner as in Comparative Example 2. The results are shown in Table 8-1 and Table 8-2. Table 8-1 shows the results of the case using the sample to be tested without dilution and Table 8-2 shows the results of the case using the sample to be tested of ten-fold dilution with the homogenizing solution.
Separately, immobilization and measurement of absorbance were performed using two specimens of the negative control (PBS containing 0.1% (W/V) BSA) attached to the kit and two specimens of the positive control (synthesized peptide of human prion protein) attached to the kit.
The results are shown in Table 9 together.
Here, in Table 8-2 and Table 9, "N.D." in the column of Comparative Example 3 indicates the case where measurement was not performed.

**Table 8-1**

| Sample No. | Example 13 | | | Comparative Example 3 |
|---|---|---|---|---|
| | mean | SD | CV% | |
| No.1 | 3.271 | 0.688 | 21% | 2.294 |
| No.2 | 3.201 | 0.578 | 18% | 3.052 |
| No.3 | 2.686 | 0.283 | 11% | 2.835 |
| No.4 | 3.330 | 0.832 | 25% | 2.501 |
| No.5 | 3.208 | 0.611 | 19% | 1.619 |
| No.6 | 3.251 | 0.694 | 21% | 2.078 |
| No.7 | 3.234 | 0.587 | 18% | 2.790 |
| No.8 | 3.115 | 0.466 | 15% | 0.935 |
| No.9 | 3.118 | 0.504 | 16% | 1.721 |
| No.10 | 3.178 | 0.554 | 17% | 0.778 |

**Table 8-2**

| Sample No. | Example 13 | | | Comparative Example 3 |
|---|---|---|---|---|
| | mean | SD | CV% | |
| No.1 | 2.427 | 0.167 | 7% | N.D. |
| No.2 | 0.792 | 0.009 | 1% | N.D. |
| No.3 | 1.684 | 0.452 | 27% | N.D. |
| No.4 | 2.719 | 0.134 | 5% | N.D. |
| No.5 | 0.556 | 0.156 | 28% | N.D. |
| No.6 | 1.854 | 0.050 | 3% | N.D. |
| No.7 | 2.709 | 0.147 | 5% | N.D. |
| No.8 | 1.124 | 0.266 | 24% | 0.091 |
| No.9 | 1.272 | 0.243 | 19% | 0.230 |
| No.10 | 1.350 | 0.313 | 23% | 0.238 |

**Table 9**

| | Sample No. | Example 13 | Comparative Example 3 |
|---|---|---|---|
| Negative Control | 1 | 0.039 | 0.024 |
| | 2 | 0.031 | 0.021 |
| | 3 | 0.034 | N.D. |
| Positive Control | 1 | 2.372 | 1.322 |
| | 2 | 2.614 | 1.267 |
| | 3 | 2.530 | N.D. |

According to the instruction of BSE kit used in Comparative Example 3, when the absorbance in the negative control is 0.15 or less, and besides the absorbance in the positive control is 1.0 or more, the detection system is judged to be reliable. As is clear from above Table 8-1 and Table 8-2, the absorbance of the negative control was 0.15 or less and an absorbance of the positive control was 1.0 or more. In the both case that measurement was performed by the detection method for PrP of the present invention (Example 13) and measurement was performed by the detection method for PrP using the conventional BSE kit (Comparative Example 3). From this fact, the detection system in the PrP detection method of the present invention can be judged to be reliable similarly as in the conventional detection method by ELISA.

Further, the cutoff value for determination of BSE infection was defined. In the case of Example 13, a value of 0.185, which was obtained by adding a mean value of the absorbance for the negative specimen performed by the method in Example 13 obtained in Table 9 above (0.035) to a mean value of absorbance for the negative specimen obtained in Example 12, i.e. (0.052) + 5SD(5 × 0.017) ≒ 0.15, was tentatively set as a cutoff value for determining BSE. When the value is applied to the results of Table 8-1 and Table 8-2, a mean value of all absorbance values in Example 13 is higher than the cutoff value, consequently all specimens could be determined as positive.
On the other hand, in Comparative Example 3, according to the instruction attached to the kit, a value of 0.233, which was obtained by adding 0.21 as described in the instruction to a mean value of absorbance for the negative control performed by the method in Comparative Example 3 obtained in Table 9 above (0.023), was tentatively set as a cutoff value for determining BSE infection. When the value is applied to the results of Table 8-1 and Table 8-2, a mean value of almost all absorbance values in Comparative Example 3 was higher than the cutoff value, consequently the specimen could be determined as positive.

However, as is clear from the results in Table 9, although the data of the negative control obtained in Example 13 were equivalent to those obtained in Comparative Example 3, the data of the positive control obtained in Example 13 were significantly higher than those obtained in Comparative Example 3, and as is clear from Table 8-1 and Table 8-2, the measured values of absorbance of the positive specimens in Example 13 was higher value than the measured values of absorbance of the specimen in Comparative Example 3.
Further, various proteins other than the abnormal PrP are contained in samples to be tested derived from animal tissues used for determination of BSE. Consequently, in order to avoid such effects, determination of BSE can be preferably made by using samples diluted as much as possible. As is clear from Table 8-2, in Example 13, even when the sample to be tested diluted ten-fold was used, the absorbance value equivalent to or higher than the value obtained by using a non-diluted specimen in Comparative Example 3 could be obtained. Consequently, according to the method for detecting abnormal PrP of the present invention, since dilution rate of sample to be tested can be increased in order to reduce an effect of coexisting substances other than abnormal PrP as much as possible, it is understood that detection of abnormal PrP with high sensitivity can be made as compared with the conventional method.

### Example 14. Comparison with detection sensitivity

### Preparation of sample to be tested

(1) Preparation of reagent solution: Following reagent solutions were prepared.
   (i) Homogenizing solution
      50 mM Tris-HCl pH 7.4 (containing 150 mM NaCl, 1 mM KCl, 1 mM EDTA, 1% (W/V) sodium deoxycholate and 1% (W/V) Sunlight SL-50 (nonionic surfactant, Sunlight K.K., Trade name)) was used.
   (ii) Protease solution
      Proteinase K (derived from Tritirachium album, Wako Pure Chemical Industries Ltd.) in an amount of 100 µg, was dissolved in 500 µL of 10 mM Tris-HCl pH 7.4 (containing 150 mM NaCl, 1 mM KCl, 1 mM EDTA, 20% (W/V) SLS and 10% (W/V) Sunlight SL-50) (final concentration of proteinase K: 200 µg/mL).
   (iii) Protein precipitation agent
      Mixed solvent of 2-butanol / methanol (5 : 1, W/W)
   (iv) Precipitate dissolving solution
      A 50 mM Tris-HCl pH 7.4 (containing 150 mM NaCl, 1 mM KCl, 1 mM EDTA, 0.005% (W/V) BSA and 2% (W/V) SDS) was used.
   (v) Normal specimen
      Small tissue pieces of the obex of medulla oblongata of bovine that had been confirmed being not infected with BSE were collected. The above homogenizing solution was added thereto so that a concentration of the small tissue pieces to the homogenizing solution became 20% (W/V), and homogenized at 2000 rpm for 30 seconds by using a multi beads shocker (Yasui Kikai Co.) in 1.3 mL of the solution for homogenization. This was used as a normal specimen.

(2) Preparation of sample to be tested
   Small tissue pieces of the cerebellum of bovine infected with BSE (positive specimen, 3 specimens) in an amount of 320 to 350 mg (wet weight) each were collected. Purified water was added thereto so that each concentration of the small pieces of tissue to purified water became 50% (W/V), and homogenized at 2000 rpm for 30 seconds by using a multi beads shocker (Yasui Kikai Co.). The homogenate frozen at -80°C was used.
   The frozen sample was thawed when the sample to be tested was prepared, and dissolved in the homogenizing solution to dilute to 2.5 fold (× 2.5) (to become 20% (W/V) sample). Subsequently, the × 2.5 diluted sample was diluted in series with the normal specimen obtained the above (1)(v) to prepare diluted samples with dilution rates of × 10, × 100, × 500, × 1000, and × 3000.
   Subsequently, 600 µL each of the thus prepared diluted sample was centrifuged at room temperature and 5000 × g, for 5 minutes to obtain supernatants.
   A protease solution (Proteinase K: 100 µg) in an amount 500 µL was added to 500 µL of the thus obtained supernatant, and mixed well. After reacting at 37°C for 10 minutes, 500 µL of the protein precipitation agent was added and mixed.
   The mixture was centrifuged at 20000 × g for 5 minutes and discarded the supernatant to obtain a precipitate.
   After dissolving the obtained precipitate by adding 60 µL of the precipitation dissolving solution, the solution was treated at 100°C for 5 minutes to inactivate proteinase K and pathogenicity of abnormal PrP.
   The thus obtained solution was used as a sample to be tested.

### Immobilization and detection of PrP

(1) Preparation of reagent solution
   (i) Immobilizing reagent solution 1
      Immobilizing reagent solution 1 containing 0.1% (W/V) SDS, 2.5% (W/V) TCA and 45% (V/V) ethanol was prepared by using purified water.
   (ii) Immobilizing reagent solution 2
      Immobilizing reagent solution 2 containing 0.1% (W/V) Tween 20, 2.5% (W/V) TCA and 45% (V/V) ethanol was prepared by using purified water.
   (iii) sample for immobilizing PrP
      A mixture of 60 µL of the sample to be tested prepared above collected in a microtube, and 600 µL of the immobilizing reagent solution 1 (final concentration of SDS 0.27% (W/V), TCA 2.3% (W/V) and ethanol 41% (V/V)) was prepared for use as an immobilization sample of PrP.
   (iv) Washing solution
      Tween 20 diluted with PBS (pH 7.4, containing 0.02% (V/V) Suraoff, (Japan EnviroChemicals, Ltd., Trade name)) to final concentration 0.05% was used.
   (v) Antibody solution
      Horse radish peroxidase labeled anti-BSE monoclonal antibody (National Institute of Animal Health, National Agriculture and Bio-oriented Research Organization) diluted to 1/500 with PBS (pH 7.4, containing 20% (W/V) glycerol and 0.1% (WN) BSA) was used.
   (vi) Coloring reagent solution
      TMB solution (Wako Pure Chemical Industries Ltd. for micro well) was used.
   (vii) Stop solution
      0.1 M HCl aqueous solution was used as the stop solution.
(2) Immobilization and detection of PrP
   Each 600 µL of the sample for immobilizing PrP prepared above was applied to two wells (duplicate for each sample, i.e. 200 µL/ each well) of a multiplate to which a PVDF membrane was set.
   The multiplate was allowed to maintain for 5 minutes and the solution in each well was suction filtered. Subsequently, 100 µL of the immobilizing reagent solution 2 was applied to each well, and suction filtered in the same manner as above. Further, 300 µL of the washing solution was applied to each well, and suction filtered in the same manner as above in order to wash the PVDF membrane of each well. This washing procedure was repeated three times. Thereafter, 50µL of the antibody solution was applied to each well and reacted at room temperature for 20 minutes. After the reaction, the procedure of applying 300 µL of the washing solution in each well and suction filtration was repeated five times to wash the PVDF membrane.
   To each well, 50 µL of the coloring reagent solution was applied and reacted at room temperature for 30 minutes in the dark place. After the reaction, the reaction mixture in the wells of multiplate was transferred to wells of 96 well ELISA plate by suction filtration. Thereafter, 50 µL each of the coloring reagent solution was applied again to the wells of multiplate to which PrP was immobilized, and the solution was transferred to the corresponding same wells of the 96 well ELISA plate by suction filtration, and the wells were washed concurrently with the reaction mixture. Then 100 µL of the stop solution was applied to each well of the 96 well ELISA plate to terminate the reaction.
   Absorbance (dominant wavelength at 450 nm, sub-wavelength at 620 nm) of the reaction mixture was measured by using a microplate reader (Molecular Devices Corp.) within 30 minutes after termination of the reaction. Mean value, SD and CV(%) of the absorbance of the duplicate measurements for each specimen are shown in Table 10. A relationship between absorbance (signal intensity) and dilution rate in each sample to be tested obtained from the positive specimen is shown in Fig. 9. In Fig. 9, -□-, -■- and--■- - show the results of the cases using sample No.1, No.2 and No.3, respectively.
Separately, immobilization of the negative control (50 mM Tris-HCl containing 0.01% (W/V) BSA and 0.02% Suraoff, pH 7.4) and the positive control (containing 50 mM Tris-HCl, pH 7.4, recombinant PrP 50 µg/mL (rPrP, supplied from Hiroshima University), 150 mM NaCl, 1 mM KCl, 1 mM EDTA, 0.01% (W/V) BSA and 2% (W/V) SDS), and measurement of absorbance were performed. The negative control and the positive control were measured in triplicate. Mean value of absorbance values obtained for each specimen is shown in Table 12.

### Comparative Example 4 (Conventional ELISA method)

PrP was detected by using Plateria (Trade Mark) BSE kit (Japan Bio-Rad Laboratories Inc.) in the same manner as in Comparative Example 1 as follows. Preparation of sample to be tested
The frozen sample of positive specimen, which was the same as that used in Example 14, was thawed and diluted with the homogenizing solution to 2.5 fold (× 2.5) (became 20% (W/W) sample). Subsequently, the × 2.5 diluted samples was diluted in series with the normal specimen obtained above to prepare diluted samples with dilution rates of × 10, ×30, × 100, ×300 and × 1000.
A solution prepared by diluting protease K (derived from Tritirachium album) attached to the kit with Reagent A solution (urea 0.12 g/mL) in an amount of 500 µL was added to 500 µL each of the thus diluted sample (without conducting the centrifugation procedure in Example 14), and reacted at 37°C for 10 minutes.
Subsequently, 500 µL of Reagent B solution (3-butanol) was added, mixed well, and centrifuged at 20000 × g for 5 minutes to obtain a precipitate.
Reagent Cl solution (urea 0.36 g/mL) was added to and dissolved the precipitate, treated at 100°C for 5 minutes to inactivate proteinase K and pathogenicity of abnormal PrP.
The thus obtained solution was used as a sample to be tested.

### Detection of PrP

The sample to be tested in an amount of 17 µL prepared above was dissolved in 83 µL of the dilution solution, and the solution was added dropwise into a 96 well ELISA plastic plate bound with anti-human PrP mouse monoclonal antibody, then allowed to stand at 37°C for 75 minutes. Subsequently, a procedure of washing the well with 350 µL of washing solution was repeated six times.
POD labeled anti-human PrP mouse monoclonal antibody solution in an amount of 100 µL was added to each well and reacted at 4°C for 60 minutes.
Each well was washed ten times with a washing solution (Tris buffer). After washing, 100 µL of the substrate coloring reagent solution (TMB solution) was applied to each well, and reacted for 30 minutes. After the reaction, 100 µL of the stop solution (0.5 M sulfuric acid) was added to each well to terminate the reaction.
Absorbance was measured with the dominant wavelength at 450 nm and sub-wavelength at 620 nm by using a microplate reader (Molecular Devices Corp.). The results are shown in Table 11. A relationship between the signal intensity and the dilution rate in each sample obtained from the positive specimen is shown in Fig. 9 together. In Fig. 9, -○-, -●- and - -●- - indicate the results obtained in samples No. 1, 2 and 3,respectively, which are the same as in Example 14.
Separately, immobilization and measurement of absorbance were performed using one specimen of the negative control (PBS containing 0.1% (W/V) BSA) attached to the kit and one specimen of the positive control (human prion protein synthetic peptide) attached to the kit.
The results are shown in Table 12 together.

**Table 10**

| Sample No. | Dilution Rate | Example 14 | | |
|---|---|---|---|---|
| | | mean | SD | CV% |
| No.1 | × 10 | 1.226 | 0.006 | 1% |
| | × 100 | 0.175 | 0.001 | 1% |
| | × 500 | 0.061 | 0.001 | 2% |
| | × 1000 | 0.054 | 0.001 | 3% |
| | × 3000 | 0.064 | 0.001 | 1% |
| No.2 | × 10 | 1.399 | 0.003 | 0% |
| | × 100 | 0.173 | 0.007 | 4% |
| | × 500 | 0.07 | 0.004 | 6% |
| | × 1000 | 0.073 | 0.002 | 3% |
| | × 3000 | 0.05 | 0.004 | 8% |
| No.3 | × 10 | 1.041 | 0.009 | 1% |
| | × 100 | 0.153 | 0.005 | 3% |
| | × 500 | 0.069 | 0.006 | 8% |
| | × 1000 | 0.056 | 0.001 | 1% |
| | × 3000 | 0.066 | 0.020 | 30% |

**Table 11**

| Sample No. | Dilution Rate | Comparative Example 4 | | |
|---|---|---|---|---|
| | | mean | SD | CV% |
| No.1 | × 10 | 0.091 | 0.008 | 9% |
| | × 30 | 0.045 | 0.002 | 4% |
| | ×100 | 0.038 | 0.000 | 0% |
| | × 300 | 0.037 | 0.002 | 5% |
| | × 1000 | 0.034 | 0.002 | 6% |
| No.2 | × 10 | 0.230 | 0.045 | 20% |
| | × 30 | 0.074 | 0.011 | 15% |
| | × 100 | 0.045 | 0.004 | 9% |
| | × 300 | 0.060 | 0.004 | 7% |
| | × 1000 | 0.041 | 0.005 | 12% |
| No.3 | × 10 | 0.238 | 0.038 | 16% |
| | × 30 | 0.058 | 0.002 | 3% |
| | × 100 | 0.046 | 0.001 | 2% |
| | × 300 | 0.037 | 0.001 | 3% |
| | ×1000 | 0.213 | 0.001 | 0% |

**Table 12**

| | Example 14 | Comparative Example 4 |
|---|---|---|
| Negative Control | 0.038 | 0.023 |
| Positive Control | 2.181 | 1.295 |

Firstly, absorbance of the negative control was 0.15 or less and absorbance of the positive control was 1.0 or more both in Example 14 and Comparative Example 4, similarly to Example 13. Consequently, the results of Example 14 can be judged reliable.
As is clear from the results of Table 10, Table 11 and Fig. 9, whereas the measurement can be performed only on a sample of about × 10 dilution by the conventional method, but measurement can be sufficiently made by the method of the present invention even with a dilution rate of × 100 or more, consequently it is found that the method of the present invention is highly sensitive for about ten times or more.

### INDUSTRIAL APPLICABILITY

According to the method for immobilizing a protein of the present invention, a protein in sample which could not easily be immobilized by the conventional solid-phase method can be immobilized in the solid-phase, and quantitative determination / detection of the protein can be performed with reduced effect of an inhibitory substance coexisting in the sample. In addition, quantitative determination of a protein which could not exactly be performed by the conventional immobilization method can also be achieved.
Further, when detection of an abnormal PrP is performed using the immobilization method, rapid and highly accurate detection and determination of prion disease (especially BSE) can be achieved.

## Claims

1. A method for immobilizing a protein to a solid-phase, comprising contacting the protein with the solid-phase having hydrophobic surface in the presence of a lower alcohol, and a halogenocarboxylic acid and/or a long chain alkyl sulfate.

2. The method according to claim 1, comprising contacting the protein with the solid-phase having hydrophobic surface in the presence of a lower alcohol, a halogenocarboxylic acid and a long chain alkyl sulfate.

3. The method according to claim 1, wherein the lower alcohol is ethanol or methanol.

4. The method according to claim 1, wherein the halogenocarboxylic acid is trichloroacetic acid (hereinafter designated as TCA) or trifluoroacetic acid (hereinafter designated as TFA).

5. The method according to claim 1, wherein the long chain alkyl sulfate is sodium dodecyl sulfate (hereinafter designated as SDS).

6. The method according to claim 1, wherein the concentration of the lower alcohol when the protein is in contact with the solid-phase having hydrophobic surface is 30 to 50% (V/V).

7. The method according to claim 1, wherein the concentration of the halogenocarboxylic acid when the protein is in contact with the solid-phase having hydrophobic surface is 0.08 to 10% (WN).

8. The method according to claim 1, wherein the concentration of the long chain alkyl sulfate when the protein is in contact with the solid-phase having hydrophobic surface is 0.1 to 1 % (WN).

9. The method according to claim 1, wherein the solid-phase is a hydrophobic membrane.

10. The method according to claim 1, wherein the lower alcohol is ethanol or methanol; the halogenocarboxylic acid is TCA or TFA; and the long chain alkyl sulfate is SDS.

11. The method according to claim 10, wherein the concentration of the lower alcohol is 30 to 50% (VN); the concentration of the halogenocarboxylic acid is 0.08 to 10% (WN); the concentration of the long chain alkyl sulfate is 0.1 to 1% (WN), when a protein is in contact with a solid-phase having hydrophobic surface; and the solid-phase is a hydrophobic membrane.

12. A method for quantitative determination of a protein, comprising contacting a protein staining solution with a solid-phase immobilized with a protein by the method of claim 1, and then determining the concentration of the protein based on a degree of color development generated thereby.

13. A method for immunoblotting comprising using the solid-phase on which the protein is immobilized by method according to claim 1 is used.

14. A method for detecting an abnormal prion protein, comprising;
immobilizing the abnormal prion protein to a solid-phase by treating a sample to be tested containing the abnormal prion protein by the method of claim 1,
reacting with an antibody capable of binding to the abnormal prion protein,
measuring an amount of an antigen - antibody complex generated thereby, and
detecting a presence of the abnormal prion protein based on the results thereof.

15. The method according to claim 14, wherein the abnormal prion protein in the sample to be tested containing the abnormal prion protein is immobilized to the solid-phase, subsequently a substance remaining unbound to the solid-phase in the sample is removed by suction filtration.

16. The method according to claim 14, further washing process of the solid-phase with a solution containing a nonionic surfactant is performed after the process of suction filtration of claim 15 is performed.

17. The method according to claim 16, wherein the solution containing the nonionic surfactant further contains a lower alcohol and a halogenocarboxylic acid.

18. The method according to claim 14, wherein the antibody is labeled with a labeling substance.

19. The method according to claim 18, wherein the method comprises a method for measuring an amount of the labeling substance of the labeled antibody bound to the abnormal prion protein, and determining an amount of the antigen - antibody complex based on the results thereof.

20. The method according to claim 19, wherein the labeling substance is an enzyme and an amount of the antigen - antibody complex is measured by an enzyme immunoassay.

21. The method according to claim 20, wherein the antigen - antibody complex of the abnormal prion protein and the enzyme labeled antibody is reacted with a substrate solution for the enzyme to generate a coloring reaction by an action of the enzyme, subsequently the substrate solution is removed by a suction filtration.

22. The method according to claim 14, wherein the sample derived from an animal tissue containing the abnormal prion protein obtained by the following processes is used as the sample to be tested:
1) a process for disrupting an animal tissue containing an abnormal PrP to be detected in the presence of a surfactant;
2) a process for removing an insoluble substance;
3) a process for decomposing a normal PrP by adding a decomposing enzyme into the supernatant;
4) a process for precipitating the abnormal PrP; and
5) a process for obtaining a solution of the precipitation after recovery of the precipitate.

23. A method for determining prion disease, comprising detecting an abnormal prion protein by the method according to claim 14, and determining prion disease based on the results thereof.

24. A reagent solution for immobilizing a protein comprising a lower alcohol, a halogenocarboxylic acid and/or a long chain alkyl sulfate.

25. The reagent solution according to claim 24, wherein the solution comprises a lower alcohol, a halogenocarboxylic acid and a long chain alkyl sulfate.

26. The reagent solution according to claim 24, wherein the lower alcohol is ethanol or methanol.

27. The reagent solution according to claim 24, wherein the halogenocarboxylic acid is TCA or TFA.

28. The reagent solution according to claim 24, wherein the long chain alkyl sulfate is SDS.

29. The reagent solution according to claim 24, wherein a concentration of the lower alcohol is 30 to 50% (V/V).

30. The reagent solution according to claim 24, wherein a concentration of the halogenocarboxylic acid is 0.1 to 10% (W/V).

31. The reagent solution according to claim 24, wherein a concentration of the long chain alkyl sulfate is 0.1 to 1% (W/V).

32. A kit for detecting an abnormal prion protein, comprising as a constituent reagent:
(1) an immobilizing reagent solution 1 containing a lower alcohol, a halogenocarboxylic acid and/or a long chain alkyl sulfate;
(2) an immobilizing reagent solution 2 containing a nonionic surfactant; and
(3) a labeled antibody capable to bind to an abnormal prion protein.

33. The kit according to claim 32, wherein the immobilizing reagent solution 2 further contains a lower alcohol and a halogenocarboxylic acid.

34. The kit according to claim 32, further comprising a substrate of the enzyme capable to generate a signal detectable by a reaction with the enzyme as a constituent reagent, when the labeled antibody is an enzyme labeled antibody.
